(19) Europäisches Patentamt
European Patent Office
Office européen des brevets

(11) EP 3 432 857 B1

(12) EUROPEAN PATENT SPECIFICATION

(45) Date of publication and mention of the grant of the patent:
**30.04.2025 Bulletin 2025/18**

(21) Application number: **17710773.7**

(22) Date of filing: **20.03.2017**

(51) International Patent Classification (IPC):
***A61K 8/39*** *(2006.01)* ***A61Q 1/04*** *(2006.01)*
***A61K 8/06*** *(2006.01)* ***A61K 8/86*** *(2006.01)*
***A61K 8/894*** *(2006.01)* ***A61K 8/49*** *(2006.01)*
***A61K 8/25*** *(2006.01)*

(52) Cooperative Patent Classification (CPC):
**A61K 8/25; A61K 8/064; A61K 8/39; A61K 8/498; A61K 8/86; A61Q 1/04;** A61K 2800/42

(86) International application number:
**PCT/EP2017/056582**

(87) International publication number:
**WO 2017/162599 (28.09.2017 Gazette 2017/39)**

(54) **COSMETIC COMPOSITION COMPRISING A WATER-SOLUBLE DYE**

KOSMETISCHE ZUSAMMENSETZUNG MIT EINEM WASSERLÖSLICHEN FARBSTOFF

COMPOSITION COSMÉTIQUE COMPRENANT UN COLORANT SOLUBLE DANS L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.03.2016 FR 1652387**
**21.03.2016 FR 1652389**

(43) Date of publication of application:
**30.01.2019 Bulletin 2019/05**

(73) Proprietor: **L'OREAL**
**75008 Paris (FR)**

(72) Inventors:
- **LE CHAUX, Laure**
  **94152 Chevilly La Rue (FR)**
- **MATTON, Marianne**
  **94152 Chevilly La Rue (FR)**
- **AUBRUN, Odile**
  **94152 Chevilly La Rue (FR)**
- **THEVENET, Ludovic**
  **94152 Chevilly La Rue (FR)**

(74) Representative: **Cabinet Nony**
**11 rue Saint-Georges**
**75009 Paris (FR)**

(56) References cited:
**US-A1- 2005 019 286** **US-A1- 2013 216 665**
**US-A1- 2014 370 062**

- **"Lip Renewal Concentrate", GNPD, February 2007 (2007-02-01), XP002761523**
- **DATABASE GNPD [online] MINTEL; February 2007 (2007-02-01), "Lip Renewal Concentrate", XP002761523, Database accession no. 663926**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 432 857 B1

## Description

The present invention relates to the field of colored cosmetic compositions, and more particularly by means of water-soluble or hydrophilic natural dyes.

There is an increasing demand from consumers for products, whether cosmetic or food products, that favor natural compounds or compounds of natural origin over synthetic compounds. This requirement of users also applies to colorants.

Within the context of the present invention, the inventors were more particularly interested in water-soluble natural dyes.

Water-soluble natural dyes essentially originate from the leaves, flowers, fruits, and roots of a broad range of plants. By way of illustration of these water-soluble dyes, the following may especially be mentioned for example: betanin (beetroot), carmine, copper chlorophyllin, and ortho-diphenol derivatives.

Among these water-soluble dyes, the natural dyes anthocyanins, belonging to the family of ortho-diphenols, are already widely used in the food industry. These compounds more particularly originate from fruits such as grapes, blackberries and plums or from vegetables such as red cabbage, radish or purple yam. Thus, they are essentially the basis of the mauve, red, blue and purple colors of flowers, fruits, leaves, seeds and roots.

As mentioned previously, these compounds give the most intense chromatic coloration when they are in a diluted form in aqueous medium, and in this regard are particularly advantageous for the cosmetics field.

Unfortunately, the coverage properties of these aqueous formulae are insufficient. In addition, the use of natural dyes, and in particular of anthocyanins in the aqueous solute state, poses problems especially in terms of stability with regard to some galenical formulations intended to incorporate them.

This is because, in the cosmetics field, a large number of cosmetic compositions conventionally employ a fatty phase. However, a lack of compatibility between the diluted form of these dyes and the fatty phase components arises. More specifically, insufficient structural stability of the corresponding compositions containing a fatty phase has been observed. This lack of stability is especially reflected by a loss of homogeneity, generally observed after storage beyond a few weeks at room temperature and beyond a few hours with heating at 90°C.

US 2014/37006 illustrates a composition 12 comprising 5 % of water, 5 % of silica aerogel particles, and 0,4 % of pearly pigment containing Mica (and) bismuth oxychloride (and) carmine.

US 2005/019286 describes in example I a lipstick comprising 2 % of ceyl/polyglyceryl-4 isostearate/hexyl laurate and dyes such as 0,9 % of Mica/titanium dioxide/carmine/methicone and 3 % of D&C Red 7#.

US 2013/216665 discloses in example I a W/O/W emulsion comprising 2 g of polyglycerol polyricinoleate emulsifier in the oily phase and 1 g of spirulina powder in 85 g of a first water phase.

The document "Lip Renewal Concentrate" with the Record ID: 663926 published by the GNPD database illustrates a lip composition comprising PEG-30 dipolyhydroxystearate and carmine.

The specific aim of the present invention is to propose a solution making it possible to overcome this lack of stability and to improve the properties of water-soluble dyes in terms of coverage.

Thus, according to a first aspect thereof, the present invention relates to a colored cosmetic composition, in particular of emulsion type, comprising, in a physiologically acceptable medium, at least one fatty phase and an aqueous phase containing at least one water-soluble natural dye or dye of natural origin chosen from anthocyanidins, anthocyanins or anthocyans, proanthocyanidins, proanthocyanins and mixture thereof, and present in a solubilized form, said composition also containing at least one effective amount of a polymeric surfactant with an HLB of between 3 and 7 and having a molecular weight of greater than 800 and/or at least one effective amount of at least one specific filler chosen from hydrophobic silica aerogel particles, wherein the aqueous phase is present in an amount ranging from 0,02 % to 20 % by weight, relative to the total weight of the composition.

In particular, the polymeric surfactant or said filler is present in an effective amount to guarantee the stability of said composition with heating for 5 hours at 95°C - 98°C at atmospheric pressure, optionally in a deflocculator.

According to a particular embodiment, the present invention relates to a colored cosmetic composition, in particular of emulsion type, comprising at least one fatty phase and from 0.02% to 20% by weight of an aqueous phase relative to the total weight of the composition, said aqueous phase containing at least one water-soluble natural dye or dye of natural origin chosen from anthocyanidins, anthocyanins or anthocyans, proanthocyanidins, proanthocyanins and mixture thereof, and present in a solubilized form, said composition also containing at least one effective amount of a polymeric surfactant with an HLB of between 3 and 7 and having a molecular weight of greater than 800.

According to a particular embodiment, the present invention relates to a colored cosmetic composition, in particular of emulsion type, comprising, in a physiologically acceptable medium, at least one fatty phase and an aqueous phase containing at least one water-soluble natural dye or dye of natural origin chosen from anthocyanidins, anthocyanins or anthocyans, proanthocyanidins, proanthocyanins and mixture thereof, and present in a solubilized form, said composition also containing at least one effective amount of at least one specific filler chosen from hydrophobic silica aerogel particles, wherein the aqueous phase is present in an amount ranging from 0,02 % to 20 % by weight, relative to the

total weight of the composition.

According to a particular embodiment, the present invention relates to a colored cosmetic composition, in particular of emulsion type, comprising, in a physiologically acceptable medium, at least one fatty phase and an aqueous phase containing at least one water-soluble natural dye or dye of natural origin chosen from anthocyanidins, anthocyanins or anthocyans, proanthocyanidins, proanthocyanins and mixture thereof, and present in a solubilized form, said composition also containing at least one effective amount of a polymeric surfactant with an HLB of between 3 and 7 and having a molecular weight of greater than 800 and at least one effective amount of at least one specific filler chosen from hydrophobic silica aerogel particles, wherein the aqueous phase is present in an amount ranging from 0,02 % to 20 % by weight, relative to the total weight of the composition.

For the purposes of the invention, a polymeric surfactant is a surfactant, the structure of which contains repeating monomer units, preferably an amount of at least 10 monomer units.

The polymeric surfactant preferably has a molecular weight greater than 1000, preferentially greater than 1500, preferentially ranging from 1500 to 50 000, preferentially ranging from 2000 to 30 000, more preferentially ranging from 2200 to 26 000, and better still from 4500 to 15 000.

Advantageously, the polymeric surfactant has an HLB value ranging from 3 to 6.5, preferably ranging from 4 to 6 and more preferentially ranging from 4.5 to 5.5.

The HLB is the ratio of the hydrophilic portion to the lipophilic portion in the molecule. The term "HLB" is well known to those skilled in the art and is described, for example, in *"The HLB system. A time-saving guide to Emulsifier Selection"* (published by ICI Americas Incorporated, 1984).

For the purposes of the present invention, "effective amount" means the amount of polymeric surfactant(s) advantageously necessary to give said composition the properties sought, in particular stability and coverage. This amount of polymeric surfactant(s) is to be considered with regard to the polymeric surfactant(s) and may consequently vary from one surfactant or mixture of surfactant(s) to another. Those skilled in the art are able, on the basis of their general knowledge, to adjust the amount of polymeric surfactant(s) necessary to give said composition the desired properties.

Thus, the polymeric surfactant is present in an effective amount to guarantee the stability of said composition with heating for 5 hours at 95°C - 98°C at atmospheric pressure, optionally in a deflocculator, especially of Rayneri type, and in particular at low speed.

It should be noted that a stable composition is a composition for which no inhomogeneity defects have been observed, especially phenomena of demixing, phase separation or the appearance of agglomerates over the course of manufacture and beyond the heat treatment detailed above. This is verified by visual observation.

According to a preferred variant, a composition comprises a content of greater than or equal to 1% by weight of polymeric surfactant(s) relative to the total weight thereof, preferably a content of greater than or equal to 1.5% by weight, better still greater than or equal to 2% by weight, even better ranging from 1% to 10% by weight, more preferentially from 1.5% to 8.5% by weight, and even more preferentially from 2% to 7.5% by weight relative to the total weight thereof.

As emerges from the examples featured below, the inventors have, against all expectations, observed that the presence of a surfactant as defined above and/or of a specific filler as defined above makes it possible to produce compositions which, although they simultaneously contain a fatty phase and a dye in aqueous solution, that is to say in the aqueous solute state, demonstrate prolonged stability over time and in terms of temperature, while demonstrating good coverage properties in terms of coloration.

Thus, the compositions according to the invention are stable.

In particular, they remain homogeneous during heating for 5 hours at a temperature of 95°C - 98°C at atmospheric pressure.

Advantageously, the specific filler according to the invention is present in an effective amount to guarantee the stability of said composition with heating for 5 hours at 95°C - 98°C at atmospheric pressure, optionally in a deflocculator, especially of Rayneri type, and in particular at low speed.

For the purposes of the present invention, "effective amount" means the amount of specific filler advantageously necessary to give said composition the properties sought, in particular stability and coverage. Those skilled in the art are able, on the basis of their general knowledge, to adjust the amount of specific filler necessary to give said composition the desired properties.

The water-soluble dye(s) of the composition according to the invention are therefore present therein essentially in a solubilized form in aqueous medium. In other words, they are not present in a significant amount, that is to say at more than 5% by weight or even at more than 1% by weight, in a particulate or even solid form in the composition.

According to a preferred variant, a composition according to the invention contains at least one anthocyanin compound as water-soluble dye.

A composition according to the invention preferably comprises a preparation step consisting of the addition of an emulsion, in particular of W/O type, and anthocyanin in powder form, pre-stabilized by an emulsifier, especially of polymeric surfactant type, for example chosen from polyglyceryl polyricinoleates. A composition according to the invention advantageously does not require the compulsory presence of an additional surfactant to guarantee the stabilization

thereof other than that originating from said emulsion of anthocyanin in powder form.

According to another aspect, the present invention relates to a cosmetic process for caring for and/or making up a keratin material, characterized in that a composition according to the invention is applied to said keratin material.

For the purposes of the present invention, the term "keratin material" is intended to cover the skin, mucous membranes such as the lips, the nails and keratin fibers, such as the eyelashes and the hair. According to the invention, the skin, in particular facial skin, the lips and the nails are particularly considered.

According to yet another aspect, the present invention relates to a process for preparing a colored cosmetic composition as defined above, comprising at least the steps consisting in:

i) providing an intermediate emulsion, for example of water-in-oil type, comprising a water-soluble natural dye or dye of natural origin chosen from anthocyanidins, anthocyanins or anthocyans, proanthocyanidins and mixture thereof, present therein in a solubilized form in aqueous medium and stabilized by at least one effective amount of a polymeric surfactant with an HLB of between 3 and 7, and having a molecular weight of greater than 800;
ii) providing a preferably colorless, for example white, cosmetic base;
iii) providing an additional amount of at least one polymeric surfactant with an HLB of between 3 and 7, and having a molecular weight of greater than 800;
iv) bringing together said intermediate emulsion from step i), said surfactant(s) from step iii), and said cosmetic base from step ii);

the bringing together of step iv) being able to be carried out in one single operation, or via successive steps, for example steps ii) and iii) being able to be carried out together, preferably during step iv), firstly the cosmetic base from step ii) and the surfactant(s) from step (iii) being brought together then secondly the intermediate emulsion being added to the cosmetic base/surfactant(s) mixture.

According to yet another particular embodiment, the surfactant(s) from step i) and the surfactant(s) from step iii) are different or of the same nature, preferably are of the same nature, and in particular chosen from polyglyceryl polyricinoleates, polydialkyl silicones with polyoxyalkylenated hydrophilic side and/or end groups, and emulsifying polymers of the fatty acid ester of polyoxyalkylenated glycol type, and mixture(s) thereof, more preferentially chosen from polyglyceryl polyricinoleates.

According to yet another aspect, the present invention relates to a process for preparing a colored cosmetic composition as defined above, comprising at least the steps consisting in:

i) providing an intermediate emulsion, for example of water-in-oil type, comprising a water-soluble natural dye or dye of natural origin chosen from anthocyanidins, anthocyanins or anthocyans, proanthocyanidins and mixture thereof, present therein in a solubilized form in aqueous medium and stabilized by at least one effective amount of a polymeric surfactant with an HLB of between 3 and 7, and having a molecular weight of greater than 800, preferably chosen from polyglyceryl polyricinoleates, polydialkyl silicones with polyoxyalkylenated hydrophilic side and/or end groups, and emulsifying polymers of the fatty acid ester of polyoxyalkylenated glycol type, and mixture(s) thereof, more preferentially chosen from polyglyceryl polyricinoleates;
ii) providing a preferably colorless, for example white, cosmetic base;
iii) providing at least one effective amount of at least one specific filler chosen from hydrophobic silica aerogel particles;
iv) bringing together said intermediate emulsion from step i), said specific filler(s) from step iii), and said cosmetic base from step ii);

the bringing together of step iv) being able to be carried out in one single operation, or via successive steps, for example steps ii) and iii) being able to be carried out together, preferably during step iv), firstly the cosmetic base from step ii) and the specific filler(s) from step iii) being brought together then secondly the intermediate emulsion being added to the cosmetic base/filler(s) mixture.

The compositions according to the invention comprise a physiologically acceptable medium.

The term "physiologically acceptable medium" is intended to denote a medium that is particularly suitable for applying a composition of the invention to keratin materials, especially the skin and more particularly facial skin, the lips and the nails. The physiologically acceptable medium is generally suited to the nature of the support onto which the product is to be applied.

A composition according to the invention may be any type of cosmetic composition such as a nail varnish, a foundation, a face powder, an eyeshadow, a concealer product, a blusher, a lipstick, a lip balm, a lip gloss, a lip pencil, an eye pencil, an eyeliner, a mascara, or else a body makeup product, a skin coloring product, or a care product such as a care cream or a tinted cream, preferably a nail varnish, a foundation or a lipstick.

A composition of the invention is especially a composition intended to be applied to a keratin material, in particular

the skin and more particularly facial skin, the lips and the nails.

Preferably, a composition according to the invention forms a product for making up keratin materials, especially the lips.

## NATURAL DYE OR DYE OF NATURAL ORIGIN

"Natural compound" means a compound that is obtained directly from the earth or the soil, or from plants or animals, via, where appropriate, one or more physical processes, for instance milling, refining, distillation, purification or filtration, or else resulting from a biotechnological process, especially resulting from cell or microbiological cultures, for example of fungi or of bacteria.

"Compound of natural origin" means a natural compound that has undergone one or more additional chemical or industrial treatments, giving rise to modifications that do not affect the essential qualities of this compound and/or a compound predominantly comprising natural constituents that may or may not have undergone transformations as indicated above. Mention may be made, as nonlimiting example of additional chemical and industrial treatment bringing about modifications which do not affect the essential qualities of a natural compound, of those allowed by the controlling bodies such as Ecocert (Reference system for biological and ecological cosmetic products, January 2003), or defined in recognized handbooks in the field, such as Cosmetics and Toiletries Magazine, 2005, Vol. 120,9:10.

According to the invention, a compound is considered to be natural or of natural origin when it is predominantly composed of natural constituents, that is to say when the weight ratio of natural constituents to non-natural constituents which make up the compound is greater than 1.

"Water-soluble dye", and more generally a "water-soluble compound" means a dye or compound which has a solubility in water, measured at 25°C, at least equal to 0.01 g/l (production of a macroscopically isotropic, transparent, colored or colorless solution).

By way of water-soluble dyes suitable for the invention, the following may especially be mentioned: water-soluble natural dyes such as, for example, anthocyanins (radish, red cabbage, purple yam, purple corn, black carrot, hibiscus, elderberries).

More particularly, the water-soluble dyes which may be used according to the invention are chosen from:

- anthocyanidins, such as cyanidin, delphinidin and petunidin;
- anthocyanins or anthocyans, such as myrtillin;
- proanthocyanidins and especially the proanthocyanidins A1, A2, B1, B2, B3 and C1;
- proanthocyanins;
  and
- the mixtures of the preceding compounds.

The water-soluble dyes which may be used according to the invention are preferably chosen from anthocyanidins, such as cyanidin, delphinidin and petunidin; anthocyanins or anthocyans, such as myrtillin; proanthocyanidins and especially the proanthocyanidins A1, A2, B1, B2, B3 and C1; proanthocyanins and mixtures of the previous compounds.

For obvious reasons, the amount of this dye or mixture of dyes present in a composition according to the invention is able to vary significantly with regard to the hue range and the chromatic intensity sought by its presence.

By way of illustration, a composition according to the invention may comprise from 0.1% to 10% by weight, and preferably from 1% to 7.5% by weight of water-soluble natural dyes or dyes of natural origin relative to the total weight thereof.

As mentioned previously, the water-soluble dye(s) are present in the aqueous solute state in a composition according to the invention. In other words, they are not present in a significant amount, that is to say at more than 5% by weight or even at more than 1% by weight, in a particulate or even solid form in the composition.

In order to obtain this type of formulation, the water-soluble dye is most commonly incorporated into the composition in the form of an aqueous solution or of an emulsion which incorporates it in its aqueous phase.

This solution or emulsion may be prepared beforehand by dilution of the solid dye in aqueous medium. Nonetheless, a certain number of commercial formulations conveying water-soluble dyes in aqueous medium, or even where appropriate formulated in the form of an emulsion, also exist.

### Anthocyanins

Structurally, anthocyanins are substituted glycosidic salts and glycosidic acyls of 2-phenylbenzopyrylium.

The basic structure of anthocyanidins consists of a chromane nucleus ($C_6$-ring A and $C_3$-ring C) and a second aromatic ring ($C_6$-ring B) in position 2, as represented in the formula below.

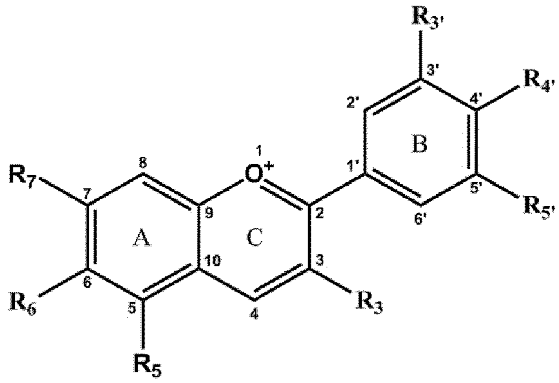

| | | | | | | | | |
|---|---|---|---|---|---|---|---|---|
| **1** | Pelargonium (Pg) | $R_3$=OH; | $R_5$=OH; | $R_6$=H; | $R_7$=OH; | $R_{3'}$=H; | $R_{4'}$=OH; | $R_{5'}$=H |
| **2** | Cyanidin (Cy) | $R_3$=OH; | $R_5$=OH; | $R_6$=H; | $R_7$=OH; | $R_{3'}$=OH; | $R_{4'}$=OH; | $R_{5'}$=H |
| **3** | Delphinidin (Dp) | $R_3$=OH; | $R_5$=OH; | $R_6$=H; | $R_7$=OH; | $R_{3'}$=OH; | $R_{4'}$=OH; | $R_{5'}$=OH |
| **4** | Peonidin (Pn) | $R_3$=OH; | $R_5$=OH; | $R_6$=H; | $R_7$=OH; | $R_{3'}$=OMe; | $R_{4'}$=OH; | $R_{5'}$=H |
| **5** | Petunidin (Pt) | $R_3$=OH; | $R_5$=OH; | $R_6$=H; | $R_7$=OH; | $R_{3'}$=OMe; | $R_{4'}$=OH; | $R_{5'}$=OH |
| **6** | Malvidin (Mv) | $R_3$=OH; | $R_5$=OH; | $R_6$=H; | $R_7$=OH; | $R_{3'}$=OMe; | $R_{4'}$=OH; | $R_{5'}$=OMe |

The different anthocyanidins differ in the number and position of the hydroxyl and/or methyl ether groups attached to their position 3, 5, 6, 7, 3', 4' and/or 5'.

Over thirty one monomeric anthocyanidins have been identified. Nonetheless, 90% of the naturally existing anthocyanins are represented by six structures (30% cyanidins (2), 22% delphinidins (3), 18% pelargonidins (1) and the final 20% made up of peonidins (4), malvidins (6) and petunidins (5)).

These six anthocyanidins are the most well-known anthocyanidins.

The color of the anthocyanidins differs with the number of hydroxyl groups attached to the molecules and more particularly those present at the ring B.

The color of the molecule changes from orange towards purple with the number of hydroxyl units present on its structure.

Glycosylation of the anthocyanidins has the effect of stimulating the red color thereof, while the presence of blue aromatic or aliphatic acyl units has an essential effect on the stability and solubility thereof.

These dyes may especially be characterized by the following CAS numbers: 134-01-1, 134-04-3, 528-53-0, 528-28-5, 643-84-5, 1429-30-7, 11029-12-2, etc., and defined by the "CTFA - International Color Handbook", Third Edition, CTFA, p. 856-857.

These natural colorants are solubilized in an aqueous phase.

According to one advantageous embodiment, the solubilized form of the anthocyanin compound in water is in the form of a water-in-oil emulsion. These emulsions are moreover stabilized by one or more surfactant(s).

Commercial formulations of this type are especially available.

By way of anthocyanin dye formulation which is suitable for the invention, the following may especially be mentioned: water-in-oil anthocyanin emulsions containing from 5% to 40% by weight, or even from 25% to 35% by weight of anthocyanins and which are stabilized by a surfactant, in particular of polyglyceryl-5 polyricinoleate type.

An example of such a formulation is especially given below.

## POLYMERIC SURFACTANT

A composition according to the invention advantageously comprises an anthocyanin introduced in emulsion form, especially W/O form, preferably stabilized by a surfactant system.

This or these surfactants preferably comprise a polymeric surfactant, preferably chosen from polyglyceryl polyricinoleates, polydialkyl silicones with polyoxyalkylenated hydrophilic side and/or end groups, and emulsifying polymers of the fatty acid ester of polyoxyalkylenated glycol type, and mixtures thereof, as described below.

The inventors have also observed the beneficial effect of the additional presence of a specific polymeric surfactant on the stabilization of the composition according to the invention. The polymeric surfactants chosen from polyglyceryl polyricinoleates, polydialkyl silicones with polyoxyalkylenated hydrophilic side and/or end groups, and emulsifying polymers of the fatty acid ester of polyoxyalkylenated glycol type are most particularly suitable for the invention.

**Polyglyceryl polyricinoleates**

According to an embodiment variant, a composition according to the invention contains at least one polyglyceryl polyricinoleate.

For the purpose of the invention, the term "polyglyceryl polyricinoleate" denotes an ester resulting from the esterification of one or more polyglycerols with at least one polyricinoleic acid.

A polyglycerol which is suitable for the invention may be chosen from the compounds of general formula (I) below:

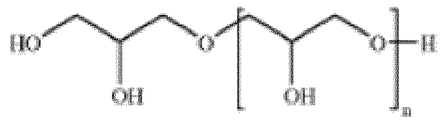

(I)

in which n represents an integer between 1 and 11, and in particular between 1 and 7, preferably between 1 and 5, even more particularly between 1 and 2.

A polyricinoleic acid which is suitable for the invention may be chosen from the compounds of general formula (II) below:

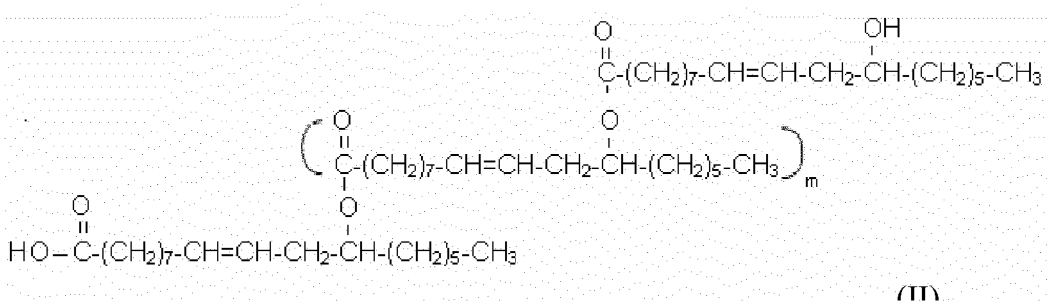

(II)

in which m represents an integer between 0 and 10, in particular between 1 and 8, and more particularly between 1 and 5.

A polyglyceryl polyricinoleate which is suitable for the invention may be a total or partial ester.

Preferably, a polyglyceryl polyricinoleate which is suitable for the invention is a partial ester.

For the purposes of the invention, the term "partial ester" is intended to denote a compound in which not all the -OH groups of the polyglycerol units have been esterified with polyricinoleic acid, in other words a polyglyceryl polyricinoleate compound comprising at least one free -OH group on the polyglycerol chain.

By way of example, a polyglyceryl polyricinoleate which is suitable for the invention may be a compound of general formula (III) below:

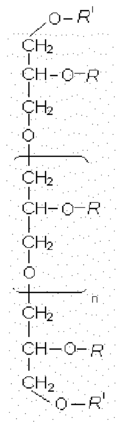

(III)

in which R and R’ represent, independently, radicals chosen from a hydrogen atom or a polyricinoleate chain, with the proviso that at least one of these R or R’ radicals is a polyricinoleate chain.

Preferably, at least one of the R or R’ groups of the polyglycerol chain is a hydrogen atom.

The polyglyceryl polyricinoleate surfactants which are suitable for the invention have an HLB (hydrophilic-lipophilic balance) of between 3 and 7.

By way of example of polyglyceryl polyricinoleates which are most particularly suitable for the invention, mention may especially be made of polyglyceryl-3 polyricinoleates, especially sold by Karlshamns under the name Akoline PGPR, by Stéarinerie Dubois Fils under the name DUB PGPR, by Dr. Straetmans under the name Dermofeel PR, by Croda under the name Crester PR, by Sasol under the name Imwitor 600 or else by Croda under the name ARLACEL 1690; polyglyceryl-6 polyricinoleates sold by Taiyo Kagaku Co. LTD under the name Sunsoft 818R and by Nikko Chemicals Co. LTD under the name Hexaglyn PR-15 or by Sakamoto Yakuhin Kogyo Co. LTD under the name S-Face CR-1001.

Within the context of the invention, mixtures of these compounds may be used.

According to one particular embodiment of the invention, the polyglyceryl polyricinoleate(s) present in the composition are chosen from polyglyceryl-3 polyricinoleate and polyglyceryl-6 polyricinoleate, and mixtures thereof. The polyglyceryl polyricinoleate(s) are preferably polyglyceryl-3 polyricinoleates.

The polyglyceryl polyricinoleate(s) may be present in the composition of the invention in an amount of active substance ranging from 1% to 10% by weight, preferably from 1.5% to 8.5% by weight and better still from 2% to 7.5% by weight relative to the total weight of the composition.

<u>**Polydialkyl silicones with polyoxyalkylenated hydrophilic side and/or end groups**</u>

The following may more particularly be mentioned as representing this second family of polymeric surfactants:

i) polydimethyl (or dialkyl) silicones with polyoxyalkylenated, polyoxyethylenated (or POE) and/or polyoxypropylenated (or PPO) side and/or end groups, comprising alkyl side groups that are more hydrophobic than the previously mentioned linear or branched $C_1$ to $C_{20}$ and preferably $C_4$ to $C_{20}$ side and/or end groups, preferably linear alkyl groups, such as lauryl or cetyl. These surfactants may also bear organosiloxane side groups.
In particular, in this first category i), mention may be made of:

- polydimethylsiloxanes containing POE side groups and alkyl side groups, especially such as cetyl PEG-PPG 10/1 dimethicone, sold under the name Abil EM 90 by Evonik Goldschmidt; and
- branched polydimethylsiloxanes containing alkyl side groups, especially such as lauryl PEG-9 polydimethyl siloxyethyl dimethicone, sold under the name KF-6038 by Shin-Etsu.

ii) polydialkyl silicones containing polyglycerol or glycerol side and/or end groups. These silicone surfactants also preferably comprise linear or branched $C_1$ to $C_{20}$ alkyl side groups, and preferably also linear alkyl groups such as lauryl or cetyl. Similarly, these silicone and glycerol surfactants may also bear organosiloxane side groups.

In particular, mention may be made, in this category ii), of:

- polydimethylsiloxanes containing polyglycerol side groups, such as polyglyceryl-3 disiloxane dimethicone, sold under the name KF-6100 by Shin-Etsu;
- branched polydimethylsiloxanes containing polyglycerol side groups, such as polyglyceryl-3 polydimethyl siloxyethyl dimethicone, sold under the name KF-6104 by Shin-Etsu; and
- branched polydimethylsiloxanes containing polyglycerol side groups and alkyl side groups, such as lauryl polyglyceryl-3 polydimethyl siloxyethyl dimethicone, sold under the name KF-6105 by Shin-Etsu.

The nonionic silicone surfactant is preferably a $C_8$-$C_{22}$ alkyl dimethicone copolyol, that is to say especially an oxypropylenated and/or oxyethylenated polymethyl ($C_8$-$C_{22}$)alkyldimethylmethylsiloxane.

The $C_8$-$C_{22}$ alkyl dimethicone copolyol is advantageously a compound of formula (IV) below:

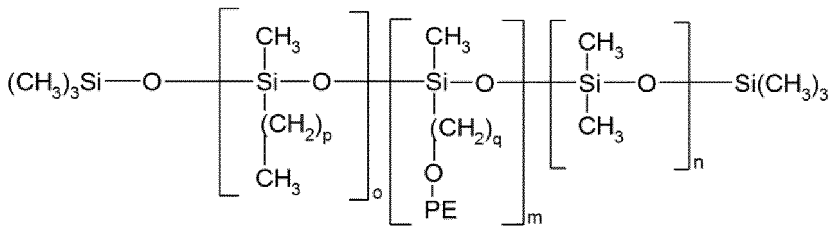

(IV)

in which:

- PE represents $(-C_2H_4O)_x-(C_3H_6O)_y-R$, R being chosen from a hydrogen atom and an alkyl radical of 1 to 4 carbon atoms, x ranging from 0 to 100 and y ranging from 0 to 80, x and y not simultaneously being 0;
- m ranging from 1 to 40;
- n ranging from 10 to 200;
- o ranging from 1 to 100;
- p ranging from 7 to 21;
- q ranging from 0 to 4;

and preferably R = H; m = 1 to 10; n = 10 to 100; o = 1 to 30; p = 15; and q = 3.

A $C_8$-$C_{22}$ alkyl dimethicone copolyol that may preferably be mentioned is cetyl dimethicone copolyol, for instance the product sold under the name Abil EM-90 by Goldschmidt.

Among the nonionic silicone surfactants, preference is given to the compound whose INCI name is cetyl PEG/PPG-10/1 dimethicone especially sold under the name Abil EM 90 by Evonik Goldschmidt.

Use may most particularly be made of alkyl dimethicone copolyols such as lauryl methicone copolyol, for example sold under the name Dow Corning 5200 Formulation Aid by Dow Corning and cetyl dimethicone copolyol, for example sold under the name Abil EM 90 by Goldschmidt, or the polyglyceryl-4 isostearate/cetyl dimethicone copolyol/hexyl laurate mixture, for example sold under the name Abil WE 09 by Goldschmidt, and cetyl dimethicone copolyol is preferably used.

Preferably, the total content of such a nonionic silicone surfactant in the composition according to the invention is between 1% and 15% by weight, preferably between 1% and 10% by weight, relative to the total weight of the composition, and preferably between 2% and 8% by weight and preferentially between 2.5% and 7% by weight.

Preferably, the total content of $C_8$-$C_{22}$ alkyl dimethicone copolyol in the composition according to the invention is between 1% and 15% by weight, preferably between 1% and 10% by weight, relative to the total weight of the composition, and preferably between 2% and 8% by weight and preferentially between 2.5% and 7% by weight.

**Emulsifying polymer of the fatty acid ester of polyoxyalkylenated glycol type**

The fatty acid ester of said polymer is preferably polyhydroxylated.

In particular, this polymer is a block polymer, preferably of ABA structure, comprising poly(hydroxylated ester) blocks and polyethylene glycol blocks.

The fatty acid ester of said emulsifying polymer as defined above generally has a chain comprising from 12 to 20 carbon atoms and preferably from 14 to 18 carbon atoms. The esters may be chosen especially from oleates, palmitates and stearates.

The polyethylene glycol blocks of said emulsifying polymer as defined above preferably comprise from 4 to 50 mol of ethylene oxide and preferably from 20 to 40 mol of ethylene oxide.

A compound that is particularly suitable for preparing the compositions of the invention is polyethylene glycol dipolyhydroxystearate with 30 OE, sold under the trade name Arlacel P 135 by ICI.

The following may also be mentioned: the PEG-30 dipolyhydroxystearate sold under the trade name Cithrol DPHS-SO-(MV) by Croda, the polyglyceryl-4 diisostearate polyhydroxystearate sebacate sold under the trade name Isolan GPS by Evonik Goldschmidt, and the dimethicone (and) dimethicone PEG-10/15 crosspolymer sold under the name KSG-210 by Shin Etsu.

Said non-ionic surfactant is preferably a $C_{10}$-$C_{18}$ fatty alkyl polyoxyether, for example a polymer of polyethylene glycol (PEG) and of $C_{10}$-$C_{18}$ fatty acids, and is preferably a polymer of polyethylene glycol (PEG) and of $C_{12}$-$C_{16}$ fatty acids comprising from 4 to 12 OE units (oxyethylene). Examples of nonionic surfactants that may especially be mentioned are laureth-4, laureth-12 and $C_{12}$-$C_{14}$ pareth-12.

The emulsifying polymer of the fatty acid ester of polyoxyalkylenated glycol type is preferably present in the compositions according to the invention in an amount ranging from 1% to 10% by weight, relative to the total weight of the composition, and more preferentially ranging from 1.5% to 5% by weight, relative to the total weight of the composition.

**SPECIFIC FILLERS**

As mentioned above, the inventors have observed the beneficial effect of the additional presence of a specific particulate filler for guaranteeing the required stabilization of a composition containing a fatty phase and containing a diluted form of one or more water-soluble dyes. These specific fillers are advantageously chosen from hydrophobic silica aerogel particles.

**Hydrophobic silica aerogel particles**

Thus, a composition according to the invention comprises hydrophobic silica aerogel particles as specific filler, which contribute to stabilizing the emulsion.

Aerogels are ultralight porous materials which were first produced by Kristler in 1932.

They are generally synthesized via a sol-gel process in liquid medium and then dried by extraction of a supercritical fluid. The supercritical fluid most commonly used is supercritical $CO_2$. Drying of this type makes it possible to avoid contraction of the pores and of the material.

Other types of drying also make it possible to obtain porous materials starting from gel, namely (i) drying by freeze drying, which consists in solidifying the gel at low temperature and in then subliming the solvent, and (ii) drying by evaporation. The materials thus obtained are referred to respectively as cryogels and xerogels. The sol-gel process and the various drying operations are described in detail in Brinker C.J. and Scherer G.W., Sol-Gel Science, New York: Academic Press, 1990.

"Hydrophobic silica" means any silica, the surface of which is treated with silylating agents, for example with halogenated silanes, such as alkylchlorosilanes, siloxanes, in particular dimethylsiloxanes, such as hexamethyldisiloxane, or silazanes, so as to functionalize the OH groups with silyl groups Si-Rn, for example trimethylsilyl groups.

Preferably, the hydrophobic aerogel particles that may be used in the present invention advantageously have a specific surface area per unit mass (SM) ranging from 500 $m^2/g$ to 1500 $m^2/g$, preferably from 600 $m^2/g$ to 1200 $m^2/g$ and better still from 600 $m^2/g$ to 800 $m^2/g$ and/or have an oil absorption capacity measured at the wet point ranging from 5 ml/g to 18 ml/g of particles, preferably from 6 ml/g to 15 ml/g and better still from 8 ml/g to 12 ml/g.

The absorption capacity, measured at the wet point and denoted Wp, corresponds to the amount of oil which it is necessary to add to 100 g of particles in order to obtain a homogeneous paste.

It is measured according to the "wet point" method or the method for determining the oil uptake of a powder according to the principle described in standard NF T 30-022. It corresponds to the amount of oil adsorbed onto the available surface of the powder and/or absorbed by the powder by measurement of the wet point, described below:
An amount m = 2 g of powder is placed on a glass plate, and the oil (isononyl isononanoate) is then added dropwise. After addition of 4 to 5 drops of oil to the powder, mixing is performed using a spatula, and addition of oil is continued until conglomerates of oil and powder have formed. From this point, the oil is added at the rate of one drop at a time and the mixture is subsequently triturated with the spatula. The addition of oil is stopped when a firm, smooth paste is obtained. This paste must be able to be spread over the glass plate without cracks or the formation of lumps. The volume Vs (expressed in ml) of oil used is then noted.

The oil uptake corresponds to the ratio Vs/m.

The hydrophobic silica aerogel particles used according to the present invention are preferably silylated silica (INCI name: silica silylate) aerogel particles.

The preparation of hydrophobic silica aerogel particles surface-modified by silylation is described more fully in US 7 470 725.

Use will be made in particular of hydrophobic silica aerogel particles surface-modified with trimethylsilyl groups, namely trimethylsiloxyl silica particles.

The hydrophobic aerogel particles that may be used in the present invention advantageously have a size, expressed as the mean diameter (D[0.5]), of less than 1500 μm, and preferably ranging from 1 μm to 30 μm, preferably from 5 μm to 25 μm, better still from 5 μm to 20 μm, and even further better still from 5 μm to 15 μm.

The specific surface area per unit mass may be determined by the nitrogen absorption method, known as the BET (Brunauer-Emmett-Teller) method, described in The Journal of the American Chemical Society, volume 60, page 309, February 1938 and corresponding to international standard ISO 5794/1 (annex D). The BET specific surface area corresponds to the total specific surface area of the particles in question.

The sizes of the aerogel particles according to the invention may be measured by static light scattering using a commercial particle size analyzer such as the MasterSizer 2000 machine from Malvern. The data are processed on the basis of the Mie scattering theory. This theory, which is exact for isotropic particles, makes it possible to determine, in the case of non-spherical particles, an "effective" particle diameter. This theory is especially described in the publication by Van de Hulst, H.C., Light Scattering by Small Particles, Chapters 9 and 10, Wiley, New York, 1957.

According to an advantageous embodiment, the hydrophobic aerogel particles used in the present invention have a specific surface area per unit mass (SM) ranging from 600 $m^2/g$ to 800 $m^2/g$ and a size, expressed as the mean diameter (D[0.5]), ranging from 5 μm to 20 μm and better still from 5 μm to 15 μm.

According to one preferred embodiment, use will be made more particularly of VM-2270, the particles of which have a mean size ranging from 5 microns to 15 microns and a specific surface area per unit mass ranging from 600 $m^2/g$ to 800 $m^2/g$.

The hydrophobic aerogel particles used in the present invention may advantageously have a tapped density $\rho$ ranging from 0.04 $g/cm^3$ to 0.10 $g/cm^3$ and preferably from 0.05 $g/cm^3$ to 0.08 $g/cm^3$.

In the context of the present invention, this density may be assessed according to the following protocol, known as the tapped density protocol:
40 g of powder are poured into a measuring cylinder and the cylinder is then placed on a Stav 2003 machine from Stampf Volumeter. The cylinder is then subjected to a series of 2500 tapping actions (this operation is repeated until the difference in volume between two consecutive tests is less than 2%); the final volume Vf of tapped powder is then measured directly on the cylinder.
The tapped density is determined by the ratio: mass (m)/Vf, in this instance 40/Vf (Vf being expressed in $cm^3$ and m in g).
According to one embodiment, the hydrophobic silica aerogel particles used in the present invention have a specific surface area per unit volume SV ranging from 5 $m^2/cm^3$ to 60 $m^2/cm^3$, preferably from 10 $m^2/cm^3$ to 50 $m^2/cm^3$ and better still from 15 $m^2/cm^3$ to 40 $m^2/cm^3$.
The specific surface area per unit volume is given by the relationship:

$$SV = SM \times \rho$$

where $\rho$ is the tapped density expressed in $g/cm^3$ and SM is the specific surface area per unit mass expressed in $m^2/g$, as defined above.
According to a preferred embodiment, the hydrophobic aerogel particles according to the invention have a specific surface area per unit mass (SM) ranging from 500 $m^2/g$ to 1500 $m^2/g$, preferably from 600 $m^2/g$ to 1200 $m^2/g$ and better still from 600 $m^2/g$ to 800 $m^2/g$, and a size expressed as the mean diameter (D[0.5]) ranging from 1 $\mu$m to 30 $\mu$m and/or an oil-absorbing capacity measured at the Wet Point ranging from 5 ml/g to 18 ml/g of particles, preferably from 6 ml/g to 15 ml/g and better still from 8 ml/g to 12 ml/g.
They may be hydrophobic silica particles (hydrophobic silica aerogels) as described in the documents WO 2013/190112, WO 2013/160362, WO 2013/190104, or WO 2013/194100. It may also be silica functionalized with anionic groups as described in the document US 2005/0192366.
As regards the preparation of hydrophobic silica aerogel particles surface-modified by silylation, reference may be made to the document US 7 470 725.
As hydrophobic silica aerogels that may be used in the invention, examples that may be mentioned include the aerogel sold under the name VM-2260 (INCI name: Silica silylate), by Dow Corning, the particles of which have a mean size of approximately 1000 microns and a specific surface area per unit mass ranging from 600 $m^2/g$ to 800 $m^2/g$.
Mention may also be made of the aerogels sold by Cabot under the references Aerogel TLD 201, Aerogel OGD 201 and Aerogel TLD 203, Enova Aerogel MT 1100 and Enova Aerogel MT 1200.
Use will be made more particularly of the aerogel sold under the name VM-2270 (INCI name: silica silylate), by Dow Corning, the particles of which have a mean size ranging from 5 microns to 15 microns and a specific surface area per unit mass ranging from 600 $m^2/g$ to 800 $m^2/g$.
A composition according to the invention preferably comprises at least 0.1% by weight of silica aerogel particles relative to the total weight thereof, or even at least 0.5% by weight, in particular at least 1% by weight, preferably at least 1.5% by weight, and even more preferentially an amount greater than 2% by weight relative to the total weight thereof.
The composition according to the invention preferably comprises from 0.5% to 15%, and more particularly from 1% to 8% by weight of silica aerogel particles relative to the total weight thereof.
According to a preferred variant, the specific filler(s) in question and the aqueous solvent medium are used in a specific filler(s) / aqueous solvent medium weight ratio at least equal to 0.5, preferably ranging from 0.6 to 16.

## AQUEOUS PHASE

A composition according to the invention comprises an aqueous phase totally or partially made up of the water carrying the water-soluble dye(s) and which is dispersed in the fatty phase therefore forming the continuous phase of the compositions according to the invention.
This aqueous phase comprises at least the water for dilution of the water-soluble dye(s), especially as detailed above.
It may also contain additional water, a floral water such as cornflower water and/or a mineral water such as Vittel water, Lucas water or La Roche Posay water and/or a spring water.
The aqueous phase, and in particular the water, provided for example by the aqueous dilution of water-soluble dye(s), may be present in a composition according to the invention in an amount ranging from 0.02% to 20% by weight, preferably ranging from 0.25% to 18% by weight and more preferentially ranging from 1% to 15% by weight relative to the total weight of the composition.
The aqueous phase of a composition according to the invention may optionally also comprise a water-soluble

solvent.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols having from 1 to 5 carbon atoms such as ethanol and isopropanol, glycols having from 2 to 8 carbon atoms such as ethylene glycol, propylene glycol, 1,3-butylene glycol and dipropylene glycol, $C_3$ and $C_4$ ketones and $C_2$-$C_4$ aldehydes.

According to another embodiment variant, the aqueous phase of a composition according to the invention may comprise at least one $C_2$-$C_{32}$ polyol.

For the purposes of the present invention, the term "polyol" should be understood as meaning any organic molecule comprising at least two free hydroxyl groups.

Preferably, a polyol in accordance with the present invention is present in liquid form at room temperature.

Such polyols may be used in an amount ranging from 0.2% to 10% by weight, preferably from 0.5% to 8% by weight and even more preferentially from 0.5% to 6% by weight of $C_2$-$C_{32}$ polyol relative to the total weight of the composition.

The polyols that are advantageously suitable for the formulation of a composition according to the present invention are those especially containing from 2 to 32 carbon atoms, preferably 3 to 16 carbon atoms and in particular from 3 to 7 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof, in particular pentylene glycol.

According to a preferred embodiment of the invention, said polyol is chosen from ethylene glycol, pentaerythritol, trimethylolpropane, propylene glycol, pentylene glycol, glycerol, polyglycerols and polyethylene glycols, and mixtures thereof.

Of course, this aqueous phase may, where appropriate, contain other hydrophilic additives, especially such as defined below.

## FATTY PHASE

As emerges from the foregoing, the cosmetic compositions according to the invention have a fatty phase.

Preferably, in the composition according to the invention, the proportion of the fatty phase may be from 99.9% to 60% by weight, preferably from 99.5% to 64% by weight and preferably from 99% to 70% by weight, relative to the total weight of the composition.

This fatty phase especially comprises an oily phase and more particularly a continuous oily phase.

This oily phase can be of very varied nature and its composition is generally adapted to the keratin target for which the corresponding cosmetic composition is intended, namely the lips, the skin or the head of hair, and to the desired purpose, namely makeup and/or care.

For the purposes of the invention, an oily phase comprises at least one oil.

The term *"oil"* means any fatty substance that is in liquid form at room temperature and atmospheric pressure.

An oily phase that is suitable for preparing the cosmetic compositions according to the invention may comprise hydrocarbon-based oils, silicone oils, fluoro oils or non-fluoro oils, or mixtures thereof.

They may be of animal, plant, mineral or synthetic origin.

For the purposes of the present invention, the term *"silicone oil"* means an oil comprising at least one silicon atom, and in particular at least one Si-O group.

The term "*fluoro oil"* means an oil comprising at least one fluorine atom.

The term *"hydrocarbon-based oil"* means an oil mainly containing hydrogen and carbon atoms.

The oils may optionally comprise oxygen, nitrogen, sulfur and/or phosphorus atoms, for example in the form of hydroxyl or acid radicals.

The oils may be volatile or non-volatile.

For the purposes of the invention, the term *"volatile oil"* means any oil that is capable of evaporating on contact with the skin in less than one hour, at room temperature and atmospheric pressure. The volatile oil is a volatile cosmetic compound, which is liquid at room temperature, especially having a nonzero vapor pressure, at room temperature and atmospheric pressure, especially having a vapor pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

## Volatile oils

The volatile oils may be hydrocarbon-based oils or silicone oils.

The hydrocarbon-based oil may be chosen from hydrocarbon-based oils having from 8 to 16 carbon atoms, and especially:

- $C_8$-$C_{16}$ branched alkanes such as $C_8$-$C_{16}$ isoalkanes of petroleum origin (also referred to as isoparaffins) such as isododecane (also referred to as 2,2,4,4,6-pentamethylheptane), isodecane, isohexadecane and, for example, the oils sold under the trade names Isopar or Permethyl;
- linear alkanes, for example such as n-dodecane ($C_{12}$) and n-tetradecane ($C_{14}$) sold by Sasol under the references PARAFOL 12-97 and PARAFOL 14-97, respectively, and also mixtures thereof, the undecane-tridecane mixture, the n-undecane ($C_{11}$) and n-tridecane ($C_{13}$) mixtures obtained in examples 1 and 2 of the application WO 2008/155059 from Cognis, and mixtures thereof;
- short-chain esters (having from 3 to 8 carbon atoms in total), such as ethyl acetate, methyl acetate, propyl acetate or n-butyl acetate;
- hydrocarbon-based oils of plant origin such as triglycerides consisting of fatty acid esters of glycerol, the fatty acids of which may have chain lengths varying from $C_4$ to $C_{24}$, these chains possibly being linear or branched, and saturated or unsaturated; these oils are especially heptanoic or octanoic acid triglycerides, or else wheatgerm oil, sunflower oil, grapeseed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion-flower oil and musk rose oil; shea butter; or else caprylic/capric acid triglycerides, for instance those sold by the company Stéarineries Dubois or those sold under the names Miglyol 810®, 812® and 818® by Dynamit Nobel;
- synthetic ethers having from 10 to 40 carbon atoms;
- linear or branched hydrocarbons of mineral or synthetic origin, such as petroleum jelly, polydecenes, hydrogenated polyisobutene such as Parleam®, squalane and liquid paraffins, and mixtures thereof;
- synthetic esters, such as oils of formula $R_1COOR_2$ in which $R_1$ represents the residue of a linear or branched fatty acid comprising from 1 to 40 carbon atoms and $R_2$ represents an especially branched hydrocarbon-based chain containing from 1 to 40 carbon atoms, provided that $R_1 + R_2 \geq 10$, for example purcellin oil (cetostearyl octanoate), isopropyl myristate, isopropyl palmitate, $C_{12}$ to $C_{15}$ alkyl benzoates, hexyl laurate, diisopropyl adipate, isononyl isononanoate, 2-ethylhexyl palmitate, isostearyl isostearate, 2-hexyldecyl laurate, 2-octyldecyl palmitate, 2-octyldodecyl myristate, alkyl or polyalkyl heptanoates, octanoates, decanoates or ricinoleates, such as propylene glycol dioctanoate; hydroxylated esters, such as isostearyl lactate, diisostearyl malate or 2-octyldodecyl lactate; polyol esters and pentaerythritol esters;
- fatty alcohols that are liquid at room temperature, with a branched and/or unsaturated carbon-based chain having from 12 to 26 carbon atoms, such as octyldodecanol, isostearyl alcohol, oleyl alcohol, 2-hexyldecanol, 2-butyloctanol and 2-undecylpentadecanol; and
- mixtures thereof.

Among the volatile hydrocarbon-based oils having from 8 to 16 carbon atoms, mention may be made especially of branched $C_8$-$C_{16}$ alkanes, for instance $C_8$-$C_{16}$ isoalkanes (also known as isoparaffins), isododecane, isodecane, isohexadecane and, for example, the oils sold under the trade names Isopar or Permethyl, branched $C_8$-$C_{16}$ esters, for instance isohexyl neopentanoate, and mixtures thereof. Preferably, the volatile hydrocarbon-based oil is chosen from volatile hydrocarbon-based oils having from 8 to 16 carbon atoms, and mixtures thereof, in particular from isododecane, isodecane and isohexadecane, and is especially isohexadecane.

Volatile silicone oils that may be mentioned include linear volatile silicone oils such as hexamethyldisiloxane, octamethyltrisiloxane, decamethyltetrasiloxane, dodecamethylpentasiloxane, tetradecamethylhexasiloxane and hexadecamethylheptasiloxane.

Volatile cyclic silicone oils that may be mentioned include hexamethylcyclotrisiloxane, octamethylcyclotetrasiloxane, decamethylcyclopentasiloxane and dodecamethylcyclohexasiloxane.

## Non-volatile oils

The non-volatile oils may, in particular, be chosen from non-volatile hydrocarbon-based, fluoro and/or silicone oils.

Non-volatile hydrocarbon-based oils that may especially be mentioned include:

- hydrocarbon-based oils of animal origin, for instance perhydrosqualene,

- hydrocarbon-based oils of plant origin, such as liquid fatty acid triglycerides comprising from 4 to 10 carbon atoms, such as heptanoic or octanoic acid triglycerides, or else, for example, sunflower oil, corn oil, soybean oil, marrow oil, grapeseed oil, sesame oil, hazelnut oil, apricot oil, macadamia oil, arara oil, castor oil, avocado oil, caprylic/capric acid triglycerides, for instance those sold by Stéarineries Dubois or those sold under the names Miglyol 810, 812 and 818 by Dynamit Nobel, jojoba oil and shea butter oil;
- synthetic ethers having from 10 to 40 carbon atoms, such as dicaprylyl ether;
- synthetic esters, for instance oils of formula $R_1COOR_2$, in which $R_1$ represents a linear or branched fatty acid residue comprising from 1 to 40 carbon atoms, and $R_2$ represents a hydrocarbon-based chain that is especially branched, containing from 1 to 40 carbon atoms provided that $R_1 + R_2 \geq 10$. The esters may be chosen especially from fatty acid alcohol esters, for instance cetostearyl octanoate, isopropyl alcohol esters such as isopropyl myristate or isopropyl palmitate, ethyl palmitate, 2-ethylhexyl palmitate, isopropyl stearate, octyl stearate, 2-octyldodecyl stearate, 2-octyldodecyl erucate, isostearyl isostearate, hydroxylated esters, such as isostearyl lactate or octyl hydroxystearate, alkyl or polyalkyl ricinoleates, hexyl laurate, fatty alcohol heptanoates, octanoates and decanoates, neopentanoic acid esters, such as isodecyl neopentanoate or isotridecyl neopentanoate, octyldodecyl neopentanoate, and isononanoic acid esters, such as isononyl isononanoate or isotridecyl isononanoate;
- polyol esters, such as propylene glycol dioctanoate, neopentyl glycol diheptanoate, and diethylene glycol diisononanoate, and pentaerythritol esters, such as dipentaerythritol tetrahydroxystearate/tetraisostearate;
- fatty alcohols that are liquid at room temperature, with a branched and/or unsaturated carbon-based chain having from 12 to 26 carbon atoms, for instance 2-octyldodecanol, isostearyl alcohol and oleyl alcohol; and
- higher $C_{12}$-$C_{22}$ fatty acids, such as oleic acid, linoleic acid and linolenic acid, and mixtures thereof.

Non-volatile silicone oils that may especially be mentioned include:

- non-phenyl silicone oils, for instance dimethicones with a viscosity greater than or equal to 10 cSt, and alkyl dimethicones such as caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes, 2-phenylethyl trimethylsiloxysilicates and trimethylpentaphenyltrisiloxane, and mixtures thereof; and also mixtures of these various oils.

A composition according to the invention may comprise from 5% to 95% by weight, better still from 5% to 40% by weight and preferably from 7% to 35% by weight of oil(s) relative to the total weight of said composition.
Advantageously, in the composition according to the invention, the oily phase(s) may comprise, or even be constituted of, a silicone oil.

**Pasty fatty substance**

A composition according to the invention may also comprise at least one pasty fatty substance.
For the purposes of the present invention, the term "pasty fatty substance" means a lipophilic fatty compound with a reversible solid/liquid change of state, which comprises, at a temperature of 23°C, a liquid fraction and a solid fraction.
In other words, the starting melting point of the pasty compound may be less than 23°C. The liquid fraction of the pasty compound measured at 23°C may represent from 9% to 97% by weight of the compound. This fraction that is liquid at 23°C preferably represents between 15% and 85% and more preferably still between 40% and 85% by weight.
Preferably, the pasty fatty substances have an end melting point of less than 60°C.
Preferably, the pasty fatty substances have a hardness of less than or equal to 6 MPa.
Preferably, the pasty fatty substances have, in the solid state, a crystalline organization, which is visible by X-ray scattering characterization.
For the purposes of the invention, the melting point corresponds to the temperature of the most endothermic peak observed on thermal analysis (DSC) as described in standard ISO 11357-3; 1999. The melting point of a pasty substance or of a wax may be measured using a differential scanning calorimeter (DSC), for example the calorimeter sold under the name DSC Q2000 by TA Instruments.
As regards the measurement of the melting point and the determination of the end melting point, the sample preparation and measurement protocols are as follows:
A sample of 5 mg of pasty fatty substance, preheated to 80°C and withdrawn with magnetic stirring using a spatula that is also heated, is placed in a hermetic aluminum capsule, or a crucible. Two tests are performed to ensure the reproducibility of the results.
The measurements are performed on the abovementioned calorimeter. The oven is flushed with nitrogen. Cooling is performed by an RCS 90 heat exchanger. The sample is then subjected to the following protocol: it is first placed at a temperature of 20°C, and then subjected to a first temperature rise passing from 20°C to 80°C, at a heating rate of

5°C/minute, then is cooled from 80°C to -80°C at a cooling rate of 5°C/minute and finally subjected to a second temperature rise passing from -80°C to 80°C at a heating rate of 5°C/minute. During the second temperature rise, the variation in the difference between the power absorbed by the empty crucible and by the crucible containing the sample of pasty substance or wax as a function of the temperature is measured. The melting point of the compound is the temperature value corresponding to the top of the peak of the curve representing the variation in the difference in power absorbed as a function of the temperature.

The end melting point corresponds to the temperature at which 95% of the sample has melted.

The liquid fraction by weight of the pasty compound at 23°C is equal to the ratio of the heat of fusion consumed at 23°C to the heat of fusion of the pasty compound.

The heat of fusion of the pasty compound is the heat consumed by the compound in order to pass from the solid state to the liquid state. The pasty compound is said to be in the solid state when all of its mass is in crystalline solid form. The pasty compound is said to be in the liquid state when all of its mass is in liquid form.

The heat of fusion of the pasty compound is equal to the integral of the entire melting curve obtained using the abovementioned calorimeter, with a temperature rise of 5°C or 10°C/minute, according to standard ISO 11357-3:1999. The heat of fusion of the pasty compound is the amount of energy required to make the compound change from the solid state to the liquid state. It is expressed in J/g.

The heat of fusion consumed at 23°C is the amount of energy absorbed by the sample to change from the solid state to the state that it has at 23°C, constituted of a liquid fraction and a solid fraction.

The liquid fraction of the pasty compound measured at 32°C preferably represents from 30% to 100% by weight of the compound, preferably from 50% to 100% and more preferably still from 60% to 100% by weight of the compound. When the liquid fraction of the pasty compound measured at 32°C is equal to 100%, the temperature of the end of the melting range of the pasty compound is less than or equal to 32°C.

The liquid fraction of the pasty compound measured at 32°C is equal to the ratio of the heat of fusion consumed at 32°C to the heat of fusion of the pasty compound. The heat of fusion consumed at 32°C is calculated in the same way as the heat of fusion consumed at 23°C.

As regards the measurement of the hardness, the sample preparation and measurement protocols are as follows:

The pasty fatty substance is placed in a mold 75 mm in diameter, which is filled to approximately 75% of its height. In order to overcome the thermal history and to control the crystallization, the mold is placed in a Vötsch VC 0018 programmable oven, where it is first placed at a temperature of 80°C for 60 minutes, then cooled from 80°C to 0°C at a cooling rate of 5°C/minute, and then left at the stabilized temperature of 0°C for 60 minutes, and then subjected to a temperature rise from 0°C to 20°C, at a heating rate of 5°C/minute, and then left at the stabilized temperature of 20°C for 180 minutes.

The compression force measurement is taken using a TA/TX2i texturometer from Swantech. The spindle used is chosen according to the texture:

- cylindrical steel spindle 2 mm in diameter for starting materials which are very rigid;
- cylindrical steel spindle 12 mm in diameter for starting materials which are not very rigid.

The measurement comprises three steps: a first step after automatic detection of the surface of the sample, where the spindle moves at a measuring speed of 0.1 mm/s, and penetrates into the pasty fatty substance to a penetration depth of 0.3 mm, the software notes the maximum force value reached; a second "relaxation" step where the spindle remains at this position for one second and the force is noted after 1 second of relaxation; finally, a third "withdrawal" step in which the spindle returns to its initial position at a speed of 1 mm/s, and the probe withdrawal energy (negative force) is noted.

The hardness value measured during the first step corresponds to the maximum compression force measured in newtons divided by the area of the texturometer cylinder expressed in $mm^2$ in contact with the pasty fatty substance. The hardness value obtained is expressed in megapascals or MPa.

The pasty fatty substance is preferably chosen from synthetic compounds and compounds of plant origin. A pasty compound may be obtained via synthesis from starting materials of plant origin.

The pasty compound is advantageously chosen from:

- lanolin and derivatives thereof;
- petroleum jelly, in particular that for which the INCI name is petrolatum and which is sold under the name Ultima White PET USP by Penreco;
- polyol ethers chosen from polyalkylene glycol pentaerythrityl ethers, fatty alcohol ethers of sugars, and mixtures thereof, the polyethylene glycol pentaerythrityl ether comprising five oxyethylene (5 OE) units (CTFA name: PEG-5 Pentaerythrityl Ether), polypropylene glycol pentaerythrityl ether comprising 5 oxypropylene (5 OP) units (CTFA name: PPG-5 Pentaerythrityl Ether) and mixtures thereof, and more especially the mixture PEG-5 Pentaerythrityl

Ether, PPG-5 Pentaerythrityl Ether and soybean oil, sold under the name Lanolide by Vevy, which is a mixture in which the constituents are in a 46/46/8 weight ratio: 46% PEG-5 pentaerythrityl ether, 46% PPG-5 pentaerythrityl ether and 8% soybean oil;

- polymeric or non-polymeric silicone compounds;
- polymeric or non-polymeric fluoro compounds;
- vinyl polymers, especially:
  - olefin homopolymers and copolymers;
  - hydrogenated diene homopolymers and copolymers;
  - linear or branched homopolymer or copolymer oligomers of alkyl (meth)acrylates preferably having a $C_8$-$C_{30}$ alkyl group;
  - homopolymer and copolymer oligomers of vinyl esters having $C_8$-$C_{30}$ alkyl groups;
  - homopolymer and copolymer oligomers of vinyl ethers having $C_8$-$C_{30}$ alkyl groups;
- liposoluble polyethers resulting from the polyetherification between one or more $C_2$-$C_{100}$ and preferably $C_2$-$C_{50}$ diols;
- esters, and/or mixtures thereof.

The pasty compound is preferably a polymer, especially a hydrocarbon-based polymer.

Among the liposoluble polyethers that are particularly preferred are copolymers of ethylene oxide and/or of propylene oxide with $C_6$-$C_{30}$ long-chain alkylene oxides, more preferably still such that the weight ratio of the ethylene oxide and/or of propylene oxide to the alkylene oxides in the copolymer is from 5:95 to 70:30. In this family, mention will be made especially of copolymers such that the long-chain alkylene oxides are arranged in blocks having an average molecular weight from 1000 to 10 000, for example a polyoxyethylene/polydodecyl glycol block copolymer such as the ethers of dodecanediol (22 mol) and of polyethylene glycol (45 OE) sold under the brand name Elfacos ST9 by AkzoNobel.

Among the esters, the following are especially preferred:

- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, preferably such as bis-diglyceryl polyacyladipate-2 sold under the brand name Softisan 649 by Sasol;
- the arachidyl propionate sold under the brand name Waxenol 801 by Alzo;
- phytosterol esters;
- fatty acid triglycerides and derivatives thereof, for instance triglycerides of fatty acid, which are especially $C_{10}$-$C_{18}$, and partially or totally hydrogenated such as those sold under the reference Softisan 100 by Sasol;
- pentaerythritol esters; and
- mixtures thereof.

Among the esters, mention may also be made of esters of a diol dimer and of a diacid dimer, where appropriate esterified on their free alcohol or acid function(s) with acid or alcohol radicals, especially dimer dilinoleate esters; such esters may be chosen especially from the esters having the following INCI nomenclature: bis-behenyl/isostearyl/phytosteryl dimer dilinoleyl dimer dilinoleate (Plandool G), phytosteryl/isostearyl/cetyl/stearyl/behenyl dimer dilinoleate (Plandool H or Plandool S), and mixtures thereof, mango butter, for example that sold under the reference Lipex 203 by AarhusKarlshamn, shea butter, in particular that for which the INCI name is Butyrospermum Parkii Butter, such as that sold under the reference Sheasoft® by AarhusKarlshamn, and mixtures thereof.

Among the pasty compounds, bis-behenyl/isostearyl/phytosteryl dimer dilinoleyl, bis(diglyceryl) poly(2-acyladipate), hydrogenated castor oil dimer dilinoleate, for example Risocast DA-L sold by Kokyu Alcohol Kogyo, and hydrogenated castor oil isostearate, for example Salacos HCIS (V-L) sold by Nisshin Oil, mango butter, shea butter, and vinylpyrrolidone/eicosene copolymers, or the mixture(s) thereof, will preferably be chosen.

According to a variant, the fatty phase may comprise vegetable butters. The vegetable butter(s) suitable for use in the invention are preferably chosen from the group comprising palm butter, avocado butter, cocoa butter, shea butter, kokum butter, mango butter, murumuru butter, coconut butter, apricot kernel butter, sal butter and urukum butter, and mixtures thereof.

A composition according to the invention may comprise one or more pasty fatty substances in a total content of greater than or equal to 2% by weight relative to the total weight of the composition, for example between 5% and 60% by weight relative to the total weight of the composition.

## Waxes

A composition according to the invention may comprise at least one wax.

For the purposes of the present invention, the term "wax" means a lipophilic fatty compound, which is solid at room temperature (25°C), with a reversible solid/liquid change of state, which has a melting point of greater than 30°C, which may be up to 200 °C, a hardness greater than 0.5 MPa, and preferably having an anisotropic crystalline organization in the solid state. By bringing the wax to its melting point, it is possible to disperse it in the oil(s) and to form a macroscopically homogeneous mixture, and on returning the temperature of the mixture to room temperature, homogeneous recrystallization of the wax from the oil(s) of the mixture is obtained.

The waxes that may be used in the invention may be hydrocarbon-based waxes and/or silicone waxes, and may be of plant, mineral, animal and/or synthetic origin, preferably hydrocarbon-based. In particular, they have a melting point of greater than 40°C and better still greater than 45°C, for example ranging from 50°C to 110°C.

As waxes that may be used in the invention, mention may be made of those generally used in the cosmetics field: they are especially of natural origin, such as beeswaxes, carnauba wax, such as the product sold especially under the name Ceruba T1 by Baerlocher, candelilla wax, ouricury wax, Japan wax, cork fiber wax or sugarcane wax, rice wax, rice bran wax, montan wax, paraffin waxes especially such as the product sold under the name Affine 56-58 Pastilles by Baerlocher, lignite wax or microcrystalline wax, ceresin or ozokerite, hydrogenated oils such as jojoba oil; synthetic waxes such as polyethylene waxes derived from the polymerization or copolymerization of ethylene and Fischer-Tropsch waxes, or alternatively fatty acid esters such as octacosanyl stearate, glycerides that are concrete at 40°C and better still at 45°C, silicone waxes such as alkyl or alkoxy dimethicones having an alkyl or alkoxy chain of 10 to 45 carbon atoms, poly(di) methylsiloxane esters that are solid at 40°C, the ester chain of which comprises at least 10 carbon atoms; and mixtures thereof.

A composition in accordance with the invention comprises a content of wax(es) of greater than or equal to 2% by weight and better still 12% by weight relative to the total weight of the composition.

As a guide, a composition according to the invention may comprise from 2% to 25% by weight, preferably from 3% to 22% by weight, better still from 4% to 21% by weight and better still from 5% to 20% by weight of wax(es), relative to the total weight of the composition.

The oily phase may also contain other fatty substances.

The other constituents that may be present in the oily phase are, for example, fatty acids or silicone resins.

These constituents may be chosen in a varied manner by those skilled in the art so as to prepare a composition having the desired properties, for example in terms of consistency or texture. Thus, in the case of a lipstick type composition, a composition according to the invention will be enriched in solid fatty substances.

## ADDITIONAL COLORANTS

The compositions in accordance with the invention may comprise at least one additional colorant.

This (or these) colorant(s) are preferably chosen from pulverulent substances, liposoluble dyes and water-soluble dyes, and mixtures thereof.

Preferably, the compositions according to the invention comprise at least one pulverulent colorant. The pulverulent colorants may be chosen from pigments and nacres, and preferably from pigments.

The pigments may be white or colored, mineral and/or organic, and coated or uncoated. Among the mineral pigments, mention may be made of metal oxides, in particular titanium dioxide, optionally surface-treated, zirconium, zinc or cerium oxides, and also iron, titanium or chromium oxides, manganese violet, ultramarine blue, chromium hydrate and ferric blue. Among the organic pigments that may be mentioned are carbon black, pigments of D & C type and lakes based on cochineal carmine or on barium, strontium, calcium or aluminum.

The nacres may be chosen from white nacreous pigments such as mica coated with titanium or with bismuth oxychloride, colored nacreous pigments such as titanium mica with iron oxides, titanium mica especially with ferric blue or chromium oxide, titanium mica with an organic pigment of the abovementioned type, and also nacreous pigments based on bismuth oxychloride.

The composition according to the invention may also comprise one or more liposoluble dyes in its fatty phase.

Examples of liposoluble dyes suitable for the invention that may be mentioned include:

- a violet organic dye, D&C Violet No. 2 K7014, chemical name: Alizurol purple SS;
- a green organic dye, D&C Green No. 6 K7016 / 90097 D&C Green 6, chemical name: quinizarine green E SS;
- a pink organic dye, D&C Red No. 21 K7061 / Suncroma D&C Red 21 C 14-032, chemical name: Eosin; and
- an orange plant dye, Betatene 30% OLV, chemical name: Carotenoids (CI. 75130-E160 A) at 30% in olive oil.

The liposoluble dyes in accordance with the invention may also be chosen from Sudan Red, D&C Red 17, β-carotene, Sudan Brown, D&C Yellow 11, D&C Orange 5, quinoline yellow and annatto.

Thus, according to a particular embodiment, a composition according to the invention also comprises at least one colorant, the colorant(s) preferably being chosen from pulverulent materials and liposoluble dyes.

These colorants may be present in an amount ranging from 0.01% to 30% by weight relative to the total weight of the composition and in particular from 0.02% to 20% by weight relative to the total weight of the composition.

## ADDITIVES

In a known way, the cosmetic composition of the invention may also contain additional adjuvants which are customary in the cosmetics field, such as liposoluble gelling agents, aqueous gelling agents, preservatives, fragrances, odorants, additional fillers, surfactants additional to those required according to the invention, liposoluble UV screening agents, bactericides, odor absorbers, inorganic or liposoluble colorants, plant extracts, salts, antioxidants, basic agents such as calcium carbonate or acidic agents such as, for example, citric or lactic acid.

The amounts of these various adjuvants are those conventionally used in the field in question, for example from 0.0001% to 20% of the total weight of the composition. These additional adjuvants can be introduced into the oily phase forming a composition according to the invention.

## GALENICS

The compositions according to the invention comprise a physiologically acceptable medium, i.e. a non-toxic medium which can be applied to human skin, and which has a pleasant appearance, odor and feel.

In particular, it will be a cosmetic composition for making up and/or caring for the skin or the hair, and more particularly the face and the lips.

More specifically, the compositions according to the invention may be makeup products, preferably of the type such as foundations, makeup bases, face powders, eyeshadows, concealer products or blushers, or else a makeup product, a product for coloring the skin, the hair or the eyelashes, and especially foundations.

Preferably, the composition according to the invention is a makeup base or a foundation.

It may also be a makeup or coloring product intended for the lips.

Likewise, the compositions according to the invention may be products for caring for the skin, the hair or the eyelashes, and more particularly the skin, and preferably a composition for protecting, treating or caring for the face, for example a day cream, night cream, anti-sun composition, protective or care body milk, after-sun milk, and more preferably a facial care composition.

In addition, the compositions under consideration according to the invention may be more or less fluid and may have the appearance of a white or colored cream, an ointment, a milk or a paste.

It is preferably solid and in particular is a lipstick stick.

Throughout the description, including the claims, the term *"comprising a"* should be understood as being synonymous with *"comprising at least one",* unless otherwise specified.

The terms *"between... and..."* and *"ranging from... to..."* should be understood as being inclusive of the limits, unless otherwise specified.

In the description and the examples, the percentages are percentages by weight, unless otherwise indicated. The percentages are thus given on a weight basis relative to the total weight of the composition. The ingredients are mixed in the order and under conditions that are easily determined by those skilled in the art.

The invention is illustrated in greater detail by the non-limiting examples presented below.

## MATERIAL AND METHOD

The compositions represented by the examples below were produced taking into consideration an anthocyanin derivative in emulsion, as prepared below, as water-soluble natural dye.

The following anthocyanin formulation was thus prepared:

| ***INCI NAME*** | ***COMMERCIAL REFERENCE*** | ***SUPPLIER*** | ***(%) BY WEIGHT*** |
|---|---|---|---|
| Red cabbage anthocyanin powder | 600008 VITIVA dd | VITIVA | 28 |
| Water | / | / | 30 |
| Coconut oil | / | RAPUNZEL NATURKOST | 31.5 |
| Polyglycerol 6 polyricinoleate | SFACE CR-1001 | SAKAMOTO | 10 |

(continued)

| INCI NAME | COMMERCIAL REFERENCE | SUPPLIER | (%) BY WEIGHT |
|---|---|---|---|
| Lactic acid | / | SIGMA-ALDRICH | 0.5 |

A W/O emulsion is prepared with the concentrated anthocyanin extracts sold by Vitiva.

The aqueous phase and the oily phase of the emulsion are prepared separately, each in their own container.

Relative to the total weight of the preparation, the aqueous phase represents 58.5% of the emulsion, comprising 28% of anthocyanins in powder form and 30.0% by weight of water. The water is acidified by addition of 0.5% lactic acid (lactic acid, ≥ 85% natural) to reach a pH of 3, which makes the anthocynanins more stable.

The oily phase is composed of a coconut oil representing 31.5% by weight of the emulsion.

These aqueous and oily phases are stabilized by means of a polyglyceryl-6 polyricinoleate emulsifier, which is introduced at the same time as the oily phase.

For this purpose, the two phases are heated to a temperature of 70°C.

When the temperature is reached, the aqueous phase is slowly added to the oily phase during constant mixing, using the high mixing head (Silverson, L5M-A) at a rotation of 7000 rpm for 10 minutes.

## Lipstick bases A to D

Four lipstick bases A to D with the following composition were prepared:

Lipstick base A:

| INCI NAME | COMMERCIAL REFERENCE | SUPPLIER | (%) BY WEIGHT |
|---|---|---|---|
| SILICA SILYLATE | DOW CORNING VM-2270 AEROGEL FINE PARTICLES | DOW CORNING | 1 |
| OCTYLDODECANOL | ISOFOL 20 N/F | SASOL | 20 |
| PETROLATUM | ULTIMA WHITE PET USP | CALUMET SPECIALTY | 15 |
| BIS-DIGLYCERYL POLYACYLADIPATE-2 | SOFTISAN 649 | CREMER OLEO | 10 |
| DIISOSTEARYL MALATE | SCHERCEMOL DISM ESTER | LUBRIZOL | 17 |
| BUTYROSPERMUM PARKII BUTTER | LIPEX SHEA | AARHUSKARLSHAM N | 5 |
| POLYBUTENE | INDOPOL H 100 | INEOS | 23 |
| POLYETHYLENE | PERFORMALENE 500-L POLYETHYLENE | NEW PHASE TECHNOLOGIES | 7 |
| HDI/TRIMETHYLOL HEXYLLACTONE CROSSPOLYMER | PLASTIC POWDER D 400 | TOSHIKI PIGMENT | 2 |

Lipstick base B:

| INCI NAME | COMMERCIAL REFERENCE | SUPPLIER | (%) BY WEIGHT |
|---|---|---|---|
| PENTAERYTHRITYL TETRA-DI-T-BUTYL HYDROXYHYDROCINNAMATE | TINOGARD TT | BASF | 0.3 |
| DISTEARDIMONIUM HECTORITE | BENTONE 38 VCG | ELEMENTIS | 0.6 |
| CERA MICROCRISTALLINA | MICROWAX HW | PARAMELT | 2.6 |
| ACETYLATED LANOLIN | ACETADEPS P80-SO-(RB) | CRODA | 8.4 |
| Cera alba | WHITE BEESWAX SP 453P | STRAHL & PITSCH | 4.2 |
| PARAFFIN (and) CERA MICROCRISTALLINA (and) SYNTHETIC WAX | BASE WAX 30540 | PARAMELT | 8 |
| OLEYL ERUCATE | CETIOL J 600 | COGNIS (BASF) | 16.9 |
| SESAMUM INDICUM OIL | SESAME SEED OIL TYPE C | NESTLE WORLD TRADE CORPORATION | 16.7 |
| LANOLIN LIQUIDA | STELLANOL 1/3 | STELLA | 33.9 |
| PPG-5 LANOLIN WAX | PURIFIED MALEIC ACID | MERCK | 8.4 |

Lipstick base C:

| INCI NAME | COMMERCIAL REFERENCE | SUPPLIER | (%) BY WEIGHT |
|---|---|---|---|
| RICINUS COMMUNIS OIL | PHARMACEUTICAL CASTOR OIL | OLVEA | 35 |
| COPERNICIA CERIFERA CERA | CARNAUBA WAX #1 FLAKES N.F. SP 63 | STRAHL & PITSCH | 6.5 |
| SIMMONDSIA CHINENSIS OIL | DW JOJOBA COLORLESS ORGANIC DESERT WHALE | DESERT WHALE | 25 |
| Cera alba | CERABEIL LOR | BAERLOCHER | 8.5 |
| OLEA EUROPAEA OIL | EXTRA VIRGIN OLIVE OIL | AARHUSKARLSHAM N | 25 |

Lipstick base D:

| INCI NAME | COMMERCIAL REFERENCE | SUPPLIER | (%) BY WEIGHT |
|---|---|---|---|
| ISOHEXADECANE | ISOHEXADECANE | INEOS | 23.8 |
| OCTYLDODECYL NEOPENTANOATE | ELEFAC I-205 | BERNEL CHEMICAL (ALZO) | 5.3 |
| BIS-BEHENYL/ISOSTEARYL/PHYTO STERYL DIMER DILINOLEYL DIMER DILINOLEATE | PLANDOOL-G | NIPPON FINE CHEMICAL | 5.7 |
| HYDROGENATED POLYISOBUTENE | PARLEAM | NOF CORPORATION | 3.4 |

(continued)

| INCI NAME | COMMERCIAL REFERENCE | SUPPLIER | (%) BY WEIGHT |
|---|---|---|---|
| VP/HEXADECENE COPOLYMER | ANTARON V 216 OR GANEX V 216 | ISP (ASHLAND) | 7.2 |
| VP/EICOSENE COPOLYMER | ANTARON V 220F OR GANEX V 220F | ISP (ASHLAND) | 1.1 |
| POLYETHYLENE | PERFORMALENE 500-L POLYETHYLENE | NEW PHASE TECHNOLOGIES | 6.1 |
| POLYETHYLENE | PERFORMALENE 400 POLYETHYLENE | NEW PHASE TECHNOLOGIES | 6.1 |
| HDI/TRIMETHYLOL HEXYLLACTONE CROSSPOLYMER | PLASTIC POWDER D 400 | TOSHIKI PIGMENT | 1.1 |
| HYDROGENATED STYRENE/METHYL STYRENE/INDENE COPOLYMER | REGALITE R1100 CG HYDROCARBON RESIN | EASTMAN CHEMICAL | 8.8 |
| ACRYLIC ACID/ISOBUTYL ACRYLATE/ISOBORNYL ACRYLATE COPOLYMER | MEXOMERE PAZ | CHIMEX | 8.8 |
| TRIMETHYLSILOXYPHENYL DIMETHICONE | BELSIL PDM 1000 | WACKER | 20.7 |
| POLYGLYCERYL-3 BEESWAX | CERA BELLINA | KOSTER KEUNEN | 1.9 |

The four lipstick bases A to D are prepared as follows:
All the ingredients are introduced into a melter brought to 90°C - 98°C. The mixture is homogenized with a Rayneri-type stirring turbine (deflocculator) until the liquor reaches a temperature of 90°C - 98°C.
After cooling, the liquor may thus be used to produce mixtures with the pigments.

## Example 1

Base A was supplemented with different surfactants at different concentrations and their respective stabilities were assessed.
For this purpose, the preferably colorless, and preferably white, lipstick substances of the base A are melted at 95°C - 98°C in a melter of jacketed heating pan type. The surfactant according to the invention is added at the amount in question relative to the total weight of the base A, and the mixture formed is homogenized using a spatula or a Rayneri deflocculator at low speed.
When the mixture is homogeneous, the anthocyanin formulation is then added, at an amount of 25% by weight relative to the total weight of the base A, and the whole mixture is homogenized using a spatula or with a Rayneri deflocculator at low speed. The formulation obtained is left with heating for 5 hours at 95°C - 98°C and with regular stirring, at least every half hour, with a spatula.
The stability is assessed visually. Defects in the stability are revealed by the presence of dark-colored agglomerates which stick to the walls. When stirring is stopped, the agglomerates sediment.
The results observed are reported in table I below. In the column indicating stability, "0" indicates that the composition is unstable, and the greater the number of "+", the more the composition is stable.

Table I

| Family | INCI name | Trade reference | Supplier | Conte nt surfac tant (%) | HLB | Molecul ar Weight | Stabili ty |
|---|---|---|---|---|---|---|---|
| Polyglyceryl polyricinoleates | PGPR | AKOLINE PGPR | AARHUSKARLSHAMN | 5 | 4 | 2500-3000 | + + + |
| Polydialkyl silicones | CETYL PEG/PPG-10/1 DIMETHICONE | ABIL EM 90 | EVONIK GOLDSCHMIDT | 2 | 5 | 13000 | + + + |
| Polydialkyl silicones | Polyglyceryl-4 isostearate + cetyl peg/ppg-dimethicone + hexyl laurate | ABIL WE 09 | EVONIK GOLDSCHMIDT | 5 | 5 | 13000 | + + |
| | | | | 7 | | | + + + |
| Emulsifying poly-mer | PEG-30 DIPOLYHYDROXYSTEA-RATE | CITHROL DPHS-SO-(MV) | CRODA | 1 | 5.5 | 5000 | + + |
| | | | | 5 | | | + + + |
| Emulsifying poly-mer | POLYGLYCERYL-4 DIISOSTEA-RATE POLYHYDROXYSTEARATE SEBACATE | ISOLAN GPS | EVONIK GOLDSCHMIDT | 2 | 5 | 5900 | + + + |
| Outside the invention | Lecithin | EMULMETIK 100 J | CARGILL | 0.5 | 4 | 758 | 0 |
| | | | | 7 | | | 0 |
| Outside the invention | Glyceryl stearate | TEGIN M PELLETS | EVONIK GOLDSCHMIDT | 2 | 3.8 | 358 | 0 |
| | | | | 7 | | | 0 |
| Outside the invention | Polyglyceryl-4 isostearate | ISOLAN GI 34 | EVONIK GOLDSCHMIDT | 2 | 5 | 580 | 0 |
| Outside the invention | PEG-10 dimethicone | KF-6017 | SHIN ETSU | 2 | 4.6 | 410 | 0 |

It emerges from these results that the compositions comprising a polymeric surfactant not in accordance with the present invention, namely a polymeric surfactant having a molecular weight of less than 800, are unstable, unlike the compositions comprising a polymeric surfactant in accordance with the present invention, which are stable.

## Example 2

Each of the bases A, B, C and D was supplemented with a particular surfactant and their respective stabilities were assessed.
The compositions are prepared according to the protocol considered in example 1. The stability is also assessed in the same way as in example 1.
The results observed are reported in table II below.

Table II

| ***Family*** | ***INCI name*** | ***Trade reference*** | ***Supplier*** | ***Conte nt surfac tant (%)*** | ***HLB*** | ***Molecul ar Weight*** | ***Base A*** | ***Base B*** | ***Base C*** | ***Base D*** |
|---|---|---|---|---|---|---|---|---|---|---|
| Emulsifying polymer | PEG-30 DIPOLYHYDROXYSTEARATE | CITHROL DPHS-SO-(MV) | CRODA | 5 | 5.5 | 5000 | + + + | + + + | + + + | + + + |

All the compositions considered are stable.

**Lipstick bases E and F**

The specific filler according to the invention is silica dimethyl silylate sold under the name Aerosil R 972 by Evonik Degussa.

Two lipstick bases E and F with the following composition were prepared.

Lipstick base E for stick

| ***INCI NAME*** | ***COMMERCIAL REFERENCE*** | ***SUPPLIER*** | ***(%) BY WEIGHT*** |
|---|---|---|---|
| EMULSION OF ANTHOCYANIN EXTRACT according to the preparation example. | | VITIVA | 15* |
| OCTYLDODECANOL | ISOFOL 20 N/F | SASOL | 16 |
| PETROLATUM | ULTIMA WHITE PET USP | CALUMET SPECIALTY | 12 |
| BIS-DIGLYCERYL POLYACYLADIPATE-2 | SOFTISAN 649 | CREMER OLEO | 8 |
| DIISOSTEARYL MALATE | SCHERCEMOL DISM ESTER | LUBRIZOL | 3 |
| BUTYROSPERMUM PARKII BUTTER | LIPEX SHEA | AARHUSKARLSHAMN | 4 |
| POLYBUTENE | INDOPOL H 100 | INEOS | 23 |
| POLYETHYLENE | PERFORMALENE 500-L POLYETHYLENE | NEW PHASE TECHNOLOGIES | 7 |
| HDI/TRIMETHYLOL HEXYLLACTONE CROSSPOLYMER | PLASTIC POWDER D 400 | TOSHIKI PIGMENT | 2 |
| * expressed by total weight of commercial product | | | |

Lipstick base F for gloss

| ***INCI NAME*** | ***COMMERCIAL REFERENCE*** | ***SUPPLIER*** | ***(%) BY WEIGHT*** |
|---|---|---|---|
| EMULSION OF ANTHOCYANIN EXTRACT according to the preparation example. | | | 15* |
| $C_{18}$-$_{36}$ ACID TRIGLYCERIDE | DUB TGI 24 | STEARINERIE DUBOIS | 17 |
| BIS-DIGLYCERYL POLYACYLADIPATE-2 | SOFTISAN 649 | CREMER OLEO | 17 |
| PENTAERYTHRITYL TETRAISOSTEARATE | CRODAMOL PTIS-LQ-(MH) | CRODA | 12 |
| TRIDECYL TRIMELLITATE | LIPONATE TDTM | LIPO CHEMICALS | 10 |

(continued)

| INCI NAME | COMMERCIAL REFERENCE | SUPPLIER | (%) BY WEIGHT |
|---|---|---|---|
| DIISOSTEARYL MALATE | SCHERCEMOL DISME-STER | LUBRIZOL | 10 |
| POLYBUTENE | INDOPOL H 100 | INEOS | 10 |
| PENTYLENE GLYCOL | 616751 HYDROLITE-5 | SYMRISE | 1 |
| * expressed by total weight of commercial product | | | |

The two lipstick bases E and F are prepared as follows:
All the ingredients are introduced into a melter brought to 90°C - 98°C. The mixture is homogenized with a Rayneri-type stirring turbine (deflocculator) until the liquor reaches a temperature of 90°C-98°C.
After cooling, the liquor may thus be used to produce mixtures with the pigments.

**Example 3**

Each of the bases E and F was supplemented with aerogel at different concentrations and their respective stabilities were assessed.
For this purpose, the colorless, and preferably white, lipstick substance of the base in question is melted at 95°C-98°C in a melter of jacketed heating pan type. The filler according to the invention is added to the amount in question and the mixture formed is homogenized using a deflocculator.
When the mixture is homogeneous, the anthocyanin formulation as prepared above is then added and the whole mixture is homogenized using a deflocculator. The formulation obtained is left with heating for 5 hours at 95°C-98°C and with gentle stirring.
The stability is assessed visually. Defects in the stability are revealed by the presence of dark-colored agglomerates which stick to the walls. When stirring is stopped, the agglomerates sediment.
The results observed are reported in table III below. In the column indicating stability, "0" indicates that the composition is unstable, and the greater the number of "+", the more the composition is stable.

TABLE III

| *Type of base* / *Content of Specific filler* | *Base E* | *Base F* |
|---|---|---|
| 0% | 0 | 0 |
| 1% | + + | + |
| 2% | + + + | + + |
| 8% | + + + | + + + |

It emerges from these results that an amount of specific filler makes it possible to stabilize the compositions according to the invention which comprise a water-soluble dye.

**Claims**

1. A colored cosmetic composition, in particular of emulsion type, comprising, in a physiologically acceptable medium, at least one fatty phase and an aqueous phase containing at least one water-soluble natural dye or dye of natural origin present therein in a solubilized form, said composition also containing at least one effective amount of a polymeric surfactant with an HLB of between 3 and 7 and having a molecular weight of greater than 800 and/or at least one effective amount of at least one specific filler chosen from hydrophobic silica aerogel particles,

wherein the aqueous phase is present in an amount ranging from 0.02% to 20% by weight, relative to the total weight of the composition, and
wherein said water-soluble natural dye or dye of natural origin is chosen from anthocyanidins; anthocyanins or anthocyans; proanthocyanidins; proanthocyanins and mixtures thereof.

2. The composition as claimed in claim 1, containing at least one effective amount of a polymeric surfactant with an HLB of between 3 and 7 and having a molecular weight of greater than 800 and at least one effective amount of at least one specific filler chosen from hydrophobic silica aerogel particles.

3. The composition as claimed in claim 1 or 2, in which the polymeric surfactant or said filler is present in an effective amount to guarantee the stability of said composition with heating for 5 hours at 95°C - 98°C at atmospheric pressure, optionally in a deflocculator.

4. The composition as claimed in either one of the preceding claims, **characterized in that** it comprises a content of greater than or equal to 1% by weight of polymeric surfactant(s) relative to the total weight thereof, preferably a content of greater than or equal to 1.5% by weight, better still greater than or equal to 2% by weight, even better ranging from 1% to 10% by weight, more preferentially from 1.5% to 8.5% by weight, and even more preferentially from 2% to 7.5% by weight relative to the total weight thereof.

5. The composition as claimed in any one of the preceding claims, in which the polymeric surfactant is chosen from polyglyceryl polyricinoleates, polydialkyl silicones with polyoxyalkylenated hydrophilic side and/or end groups, and emulsifying polymers of the fatty acid ester of polyoxyalkylenated glycol type.

6. The composition as claimed in any one of the preceding claims, in which the polymeric surfactant is chosen from polyglyceryl polyricinoleates, preferably from polyglyceryl-3 polyricinoleate, polyglyceryl-6 polyricinoleate, and mixtures thereof, and is more preferentially polyglyceryl-3 polyricinoleate.

7. The composition as claimed in any one of claims 1 to 5, in which the polymeric surfactant is chosen from alkyl dimethicone copolyols such as lauryl methicone copolyol and cetyl dimethicone copolyol, and the polyglyceryl-4 isostearate/cetyl dimethicone copolyol/hexyl laurate mixture, and is preferably cetyl dimethicone copolyol.

8. The composition as claimed in any one of claims 1 to 5, in which the polymeric surfactant is chosen from $C_{10}$-$C_{18}$ fatty alkyl polyoxyethers, for example a polymer of polyethylene glycol (PEG) and of $C_{10}$-$C_{18}$ fatty acids, and is preferably a polymer of polyethylene glycol (PEG) and of $C_{12}$-$C_{16}$ fatty acids comprising from 4 to 12 OE units (oxyethylene).

9. The composition as claimed in any one of the preceding claims, in which said specific filler(s) are present in a specific filler(s) / aqueous solvent medium weight ratio at least equal to 0.5, preferably ranging from 0.6 to 16.

10. The composition as claimed in any one of the preceding claims, in which the hydrophobic silica aerogel particles are trimethylsiloxyl silica particles.

11. The composition as claimed in any one of the preceding claims, in which said water-soluble dye is an anthocyanin.

12. The composition as claimed in any one of the preceding claims, **characterized in that** it comprises from 0.1% to 10% by weight, preferably from 1% to 7.5% by weight of water-soluble natural dyes or dyes of natural origin relative to the total weight thereof.

13. A cosmetic process for caring for and/or making up a keratin material, **characterized in that** a composition as claimed in any one of the preceding claims is applied to said keratin material.

14. A process for preparing a colored cosmetic composition as claimed in any one of claims 1 to 12, comprising at least the steps consisting in:

i) providing an intermediate emulsion, for example of water-in-oil type, comprising a water-soluble natural dye or dye of natural origin, present therein in a solubilized form in aqueous medium and stabilized by at least one effective amount of a polymeric surfactant with an HLB of between 3 and 7, and having a molecular weight of greater than 800;
ii) providing a preferably colorless, for example white, cosmetic base;

iii) providing an additional amount of at least one polymeric surfactant with an HLB of between 3 and 7, and having a molecular weight of greater than 800;
iv) bringing together said intermediate emulsion from step i), said surfactant(s) from step iii), and said cosmetic base from step ii);
the bringing together of step iv) being able to be carried out in one single operation, or via successive steps, for example steps ii) and iii) being able to be carried out together, preferably during step iv), firstly the cosmetic base from step ii) and the surfactant(s) from step iii) being brought together then secondly the intermediate emulsion being added to the cosmetic base/surfactant(s) mixture,
wherein said water-soluble natural dye or dye of natural origin is chosen from anthocyanidins; anthocyanins or anthocyans; proanthocyanidins; proanthocyanins and mixtures thereof.

**15.** The preparation process as claimed in claim 14, in which the surfactant(s) from step i) and the surfactant(s) from step iii) are different or of the same nature, preferably are of the same nature, and in particular chosen from polyglyceryl polyricinoleates, polydialkyl silicones with polyoxyalkylenated hydrophilic side and/or end groups, and emulsifying polymers of the fatty acid ester of polyoxyalkylenated glycol type, and mixture(s) thereof, more preferentially chosen from polyglyceryl polyricinoleates.

**16.** A process for preparing a colored cosmetic composition as claimed in any one of claims 1 to 12, comprising at least the steps consisting in:

i) providing an intermediate emulsion, for example of water-in-oil type, comprising a water-soluble natural dye or dye of natural origin, present therein in a solubilized form in aqueous medium and stabilized by at least one effective amount of a polymeric surfactant with an HLB of between 3 and 7, and having a molecular weight of greater than 800, preferably chosen from polyglyceryl polyricinoleates, polydialkyl silicones with polyoxyalkylenated hydrophilic side and/or end groups, and emulsifying polymers of the fatty acid ester of polyoxyalkylenated glycol type, and mixture(s) thereof, more preferentially chosen from polyglyceryl polyricinoleates;
ii) providing a preferably colorless, for example white, cosmetic base;
iii) providing at least one effective amount of at least one specific filler chosen from hydrophobic silica aerogel particles;
iv) bringing together said intermediate emulsion from step i), said specific filler(s) from step iii), and said cosmetic base from step ii);
the bringing together of step iv) being able to be carried out in one single operation, or via successive steps, for example steps ii) and iii) being able to be carried out together, preferably during step iv), firstly the cosmetic base from step ii) and the specific filler(s) from step iii) being brought together then secondly the intermediate emulsion being added to the cosmetic base/filler(s) mixture,
wherein said water-soluble natural dye or dye of natural origin is chosen from anthocyanidins; anthocyanins or anthocyans; proanthocyanidins; proanthocyanins and mixtures thereof.

## Patentansprüche

**1.** Gefärbte kosmetische Zusammensetzung, insbesondere vom Emulsionstyp, die in einem physiologisch unbedenklichen Medium mindestens eine Fettphase und eine wässrige Phase, die mindestens einen darin in solubilisierter Form vorliegenden wasserlöslichen natürlichen Farbstoff oder Farbstoff natürlichen Ursprungs enthält, umfasst, wobei die Zusammensetzung ferner mindestens eine wirksame Menge eines polymeren Tensids mit einem HLB-Wert zwischen 3 und 7 und einem Molekulargewicht von mehr als 800 und/oder mindestens eine wirksame Menge mindestens eines spezifischen Füllstoffs, der aus hydrophoben Siliciumdioxid-Aerogelteilchen ausgewählt ist, enthält,

wobei die wässrige Phase in einer Menge in einem Bereich von 0,02 bis 20 Gew.-%, bezogen auf das Gesamtgewicht der Zusammensetzung, vorliegt und
wobei der wasserlösliche natürliche Farbstoff oder Farbstoff natürlichen Ursprungs aus Anthocyanidinen; Anthocyaninen oder Anthocyanen; Proanthocyanidinen; Proanthocyaninen und Mischungen davon ausgewählt ist.

**2.** Zusammensetzung nach Anspruch 1, enthaltend mindestens eine wirksame Menge eines polymeren Tensids mit einem HLB-Wert zwischen 3 und 7 und einem Molekulargewicht von mehr als 800 und mindestens eine wirksame Menge mindestens eines spezifischen Füllstoffs, der aus hydrophoben Siliciumdioxid-Aerogelteilchen ausgewählt

ist.

3. Zusammensetzung nach Anspruch 1 oder 2, wobei das polymere Tensid oder der Füllstoff in einer wirksamen Menge zur Gewährleistung der Stabilität der Zusammensetzung bei 5 Stunden Erhitzen auf 95 °C - 98 °C bei Normaldruck, gegebenenfalls in einem Entflocker, vorliegt.

4. Zusammensetzung nach einem der vorhergehenden Ansprüche, **dadurch gekennzeichnet, dass** sie einen Gehalt größer oder gleich 1 Gew.-% polymeres Tensid bzw. polymere Tenside, bezogen auf ihr Gesamtgewicht, vorzugsweise einen Gehalt größer oder gleich 1,5 Gew.-%, besser größer oder gleich 2 Gew.-%, noch besser im Bereich von 1 bis 10 Gew.-%, weiter bevorzugt von 1,5 bis 8,5 Gew.-% und noch weiter bevorzugt 2 bis 7,5 Gew.-%, bezogen auf ihr Gesamtgewicht, umfasst.

5. Zusammensetzung nach einem der vorhergehenden Ansprüche, wobei das polymere Tensid aus Polyglycerylpolyricinoleaten, Polydialkylsilikonen mit polyoxyalkylenierten hydrophilen Seiten- und/oder Endgruppen und emulgierenden Polymeren des Typs Fettsäureester von polyoxyalkyleniertem Glykol ausgewählt ist.

6. Zusammensetzung nach einem der vorhergehenden Ansprüche, wobei das polymere Tensid aus Polyglycerylpolyricinoleaten, vorzugsweise aus Polyglyceryl-3-polyricinoleat, Polyglyceryl-6-polyricinoleat und Mischungen davon, ausgewählt ist und weiter bevorzugt Polyglyceryl-3-polyricinoleat ist.

7. Zusammensetzung nach einem der Ansprüche 1 bis 5, wobei das polymere Tensid aus Alkyldimethiconcopolyolen wie Laurylmethiconcopolyol und Cetyldimethiconcopolyol und der Mischung Polyglyceryl-4-isostearat/Cetyldimethiconcopolyol/Hexyllaurat ausgewählt ist und vorzugsweise Cetyldimethiconcopolyol ist.

8. Zusammensetzung nach einem der Ansprüche 1 bis 5, wobei das polymere Tensid aus $C_{10}$-$C_{18}$-Fettalkylpolyoxyethern, beispielsweise einem Polymer von Polyethylenglykol (PEG) und von $C_{10}$-$C_{18}$-Fettsäuren, ausgewählt ist und vorzugsweise ein Polymer von Polyethylenglykol (PEG) und von $C_{12}$-$C_{16}$-Fettsäuren mit 4 bis 12 OE-Einheiten (Oxyethylen) ist.

9. Zusammensetzung nach einem der vorhergehenden Ansprüche, wobei der spezifische Füllstoff bzw. die spezifischen Füllstoffe in einem Gewichtsverhältnis von spezifischem Füllstoff bzw. spezifischen Füllstoffen zu wässrigem Lösungsmittelmedium von mindestens 0,5, vorzugsweise im Bereich von 0,6 bis 16, vorliegt bzw. vorliegen.

10. Zusammensetzung nach einem der vorhergehenden Ansprüche, wobei es sich bei den hydrophoben Siliciumdioxid-Aerogelteilchen um Trimethylsiloxylsiliciumdioxidteilchen handelt.

11. Zusammensetzung nach einem der vorhergehenden Ansprüche, wobei es sich bei dem wasserlöslichen Farbstoff um ein Anthocyanin handelt.

12. Zusammensetzung nach einem der vorhergehenden Ansprüche, **dadurch gekennzeichnet, dass** sie 0,1 bis 10 Gew.-%, vorzugsweise 1 bis 7,5 Gew.-%, wasserlösliche natürliche Farbstoffe oder Farbstoffe natürlichen Ursprungs, bezogen auf ihr Gesamtgewicht, umfasst.

13. Kosmetisches Verfahren zum Pflegen und/oder Schminken eines Keratinmaterials, **dadurch gekennzeichnet, dass** man auf das Keratinmaterial eine Zusammensetzung nach einem der vorhergehenden Ansprüche aufbringt.

14. Verfahren zur Herstellung einer gefärbten kosmetischen Zusammensetzung nach einem der Ansprüche 1 bis 12, umfassend mindestens die Schritte, die aus Folgendem bestehen:

i) Bereitstellen einer Zwischenemulsion, beispielsweise vom Wasser-in-Öl-Typ, umfassend einen darin in einer solubilisierten Form in wässrigem Medium vorliegenden und durch mindestens eine wirksame Menge eines polymeren Tensids mit einem HLB-Wert zwischen 3 und 7 und einem Molekulargewicht von mehr als 800 stabilisierten wasserlöslichen natürlichen Farbstoff oder Farbstoff natürlichen Ursprungs;
ii) Bereitstellen einer vorzugsweise farblosen, beispielsweise weißen, kosmetischen Grundlage;
iii) Bereitstellen einer zusätzlichen Menge mindestens eines polymeren Tensids mit einem HLB-Wert zwischen 3 und 7 und einem Molekulargewicht von mehr als 800;
iv) Zusammenbringen der Zwischenemulsion aus Schritt i), des Tensids bzw. der Tenside aus Schritt iii) und der kosmetischen Grundlage aus Schritt ii);

wobei das Zusammenbringen von Schritt iv) in einem einzigen Arbeitsgang oder über aufeinanderfolgende Schritte durchgeführt werden kann, wobei beispielsweise die Schritte ii) und iii) zusammen, vorzugsweise während Schritt iv), durchgeführt werden können, wobei zunächst die kosmetische Grundlage aus Schritt ii) und das Tensid bzw. die Tenside aus Schritt iii) zusammengebracht werden und dann zweitens die Zwischenemulsion zu der Mischung von kosmetischer Grundlage und Tensid bzw. Tensiden gegeben wird,
wobei der wasserlösliche natürliche Farbstoff oder Farbstoff natürlichen Ursprungs aus Anthocyanidinen; Anthocyaninen oder Anthocyanen; Proanthocyanidinen; Proanthocyaninen und Mischungen davon ausgewählt ist.

**15.** Herstellungsverfahren nach Anspruch 14, bei dem das Tensid bzw. die Tenside von Schritt i) und das Tensid bzw. die Tenside von Schritt iii) verschieden oder gleichartig, vorzugsweise gleichartig, sind und insbesondere aus Polyglycerylpolyricinoleaten, Polydialkylsilikonen mit polyoxyalkylenierten hydrophilen Seiten- und/oder Endgruppen und emulgierenden Polymeren des Typs Fettsäureester von polyoxyalkyleniertem Glykol und einer Mischung bzw. Mischungen davon ausgewählt sind und insbesondere aus Polyglycerylpolyricinoleaten ausgewählt sind.

**16.** Verfahren zur Herstellung einer gefärbten kosmetischen Zusammensetzung nach einem der Ansprüche 1 bis 12, umfassend mindestens die Schritte, die aus Folgendem bestehen:

i) Bereitstellen einer Zwischenemulsion, beispielsweise vom Wasser-in-Öl-Typ, umfassend einen darin in einer solubilisierten Form in wässrigem Medium vorliegenden und durch mindestens eine wirksame Menge eines polymeren Tensids, mit einem HLB-Wert zwischen 3 und 7 und einem Molekulargewicht von mehr als 800, das vorzugsweise aus Polyglycerylpolyricinoleaten, Polydialkylsilikonen mit polyoxyalkylenierten hydrophilen Seiten- und/oder Endgruppen und emulgierenden Polymeren des Typs Fettsäureester von polyoxyalkyleniertem Glykol und einer Mischung bzw. Mischungen davon ausgewählt ist und weiter bevorzugt aus Polyglycerylpolyricinoleaten ausgewählt ist, stabilisierten wasserlöslichen natürlichen Farbstoff oder Farbstoff natürlichen Ursprungs;
ii) Bereitstellen einer vorzugsweise farblosen, beispielsweise weißen, kosmetischen Grundlage;
iii) Bereitstellen mindestens einer wirksamen Menge mindestens eines spezifischen Füllstoffs, der aus hydrophoben Siliciumdioxid-Aerogelteilchen ausgewählt ist;
iv) Zusammenbringen der Zwischenemulsion aus Schritt i), des spezifischen Füllstoffs bzw. der spezifischen Füllstoffe aus Schritt iii) und der kosmetischen Grundlage aus Schritt ii);
wobei das Zusammenbringen von Schritt iv) in einem einzigen Arbeitsgang oder über aufeinanderfolgende Schritte durchgeführt werden kann, wobei beispielsweise die Schritte ii) und iii) zusammen, vorzugsweise während Schritt iv), durchgeführt werden können, wobei zunächst die kosmetische Grundlage aus Schritt ii) und der spezifische Füllstoff bzw. die spezifischen Füllstoffe aus Schritt iii) zusammengebracht werden und dann zweitens die Zwischenemulsion zu der Mischung von kosmetischer Grundlage und dem spezifischen Füllstoff bzw. den spezifischen Füllstoffen gegeben wird,
wobei der wasserlösliche natürliche Farbstoff oder Farbstoff natürlichen Ursprungs aus Anthocyanidinen; Anthocyaninen oder Anthocyanen; Proanthocyanidinen; Proanthocyaninen und Mischungen davon ausgewählt ist.

## Revendications

**1.** Composition cosmétique colorée, notamment de type émulsion, comprenant, dans un milieu physiologiquement acceptable, au moins une phase grasse et une phase aqueuse contenant au moins un colorant naturel hydrosoluble ou colorant d'origine naturelle présent dans celle-ci sous une forme solubilisée, ladite composition contenant en outre au moins une quantité efficace d'un tensioactif polymérique doté d'un HLB compris entre 3 et 7 et ayant un poids moléculaire supérieur à 800 et/ou au moins une quantité efficace d'au moins une charge spécifique choisie parmi des particules d'aérogel de silice hydrophobe,

dans laquelle la phase aqueuse est présente en une quantité allant de 0,02 % à 20 % en poids, par rapport au poids total de la composition, et
dans lequel ledit colorant naturel hydrosoluble ou colorant d'origine naturelle est choisi parmi les anthocyanidines ; les anthocyanines ou les anthocyanes ; les proanthocyanidines ; les proanthocyanines et leurs mélanges.

**2.** Composition selon la revendication 1, contenant au moins une quantité efficace d'un tensioactif polymérique ayant un HLB compris entre 3 et 7 et ayant un poids moléculaire supérieur à 800 et au moins une quantité efficace d'au moins

une charge spécifique choisie parmi des particules d'aérogel de silice hydrophobe.

**3.** Composition selon la revendication 1 ou 2, dans laquelle le tensioactif polymérique ou ladite charge est présent(e) en une quantité efficace pour garantir la stabilité de ladite composition avec chauffage pendant 5 heures à 95 °C - 98 °C à pression atmosphérique, éventuellement sous défloculeuse.

**4.** Composition selon l'une quelconque des revendications précédentes, **caractérisée en ce qu'**elle comprend une teneur supérieure ou égale à 1 % en poids de tensioactif(s) polymérique(s) par rapport au poids total de celui-ci, de préférence une teneur supérieure ou égale à 1,5 % en poids, mieux encore supérieure ou égale à 2 % en poids, encore mieux allant de 1 % à 10 % en poids, plus préférablement de 1,5 % à 8,5 % en poids, et encore plus préférablement de 2 % à 7,5 % en poids par rapport à leur poids total.

**5.** Composition selon l'une quelconque des revendications précédentes, dans laquelle le tensioactif polymérique est choisi parmi les polyricinoléates de polyglycéryle, les polydialkylsilicones à groupes latéraux et/ou terminaux hydrophiles polyoxyalkylénés, et les polymères émulsifiants du type ester d'acide gras de glycol polyoxyalkyléné.

**6.** Composition selon l'une quelconque des revendications précédentes, dans laquelle le tensioactif polymérique est choisi parmi les polyricinoléates de polyglycéryle, de préférence parmi le polyricinoléate de polyglycéryle-3, le polyricinoléate de polyglycéryle-6 et leurs mélanges, et est plus préférablement le polyricinoléate de polyglycéryle-3.

**7.** Composition selon l'une quelconque des revendications 1 à 5, dans laquelle le tensioactif polymérique est choisi parmi les alkyldiméthicone copolyols tels que le laurylméthicone copolyol et le cétyldiméthicone copolyol, et le mélange isostéarate de polyglycéryle-4/cétyldiméthicone copolyol/laurate d'hexyle, et est de préférence un cétyldiméthicone copolyol.

**8.** Composition selon l'une quelconque des revendications 1 à 5, dans laquelle le tensioactif polymérique est choisi parmi les alkylpolyoxyéthers gras en $C_{10}$-$C_{18}$, par exemple un polymère de polyéthylèneglycol (PEG) et d'acides gras en $C_{10}$-$C_{18}$, et est de préférence un polymère de polyéthylène glycol (PEG) et d'acides gras en $C_{12}$-$C_{16}$ comprenant de 4 à 12 motifs d'OE (oxyéthylène).

**9.** Composition selon l'une quelconque des revendications précédentes, dans laquelle ladite ou lesdites charges spécifiques sont présentes en un rapport pondéral charge(s) spécifique(s) / milieu solvant aqueux au moins égal à 0,5, de préférence allant de 0,6 à 16.

**10.** Composition selon l'une quelconque des revendications précédentes, dans laquelle les particules d'aérogel de silice hydrophobe sont des particules de triméthylsiloxylsilice.

**11.** Composition selon l'une quelconque des revendications précédentes, dans laquelle ledit colorant hydrosoluble est une anthocyanine.

**12.** Composition selon l'une quelconque des revendications précédentes, **caractérisée en ce qu'**elle comprend de 0,1 % à 10 % en poids, de préférence de 1 % à 7,5 % en poids de colorants naturels solubles dans l'eau ou de colorants d'origine naturelle par rapport à leur poids total.

**13.** Procédé cosmétique de soin et/ou de maquillage d'une matière kératinique, **caractérisé en ce qu'**une composition selon l'une quelconque des revendications précédentes est appliquée sur ladite matière kératinique.

**14.** Procédé de préparation d'une composition cosmétique colorée selon l'une quelconque des revendications 1 à 12, comprenant au moins les étapes consistant en :

i) la fourniture d'une émulsion intermédiaire, par exemple de type eau dans huile, comprenant un colorant naturel hydrosoluble ou colorant d'origine naturelle, présent dans celle-ci sous une forme solubilisée en milieu aqueux et stabilisé par au moins une quantité efficace d'un tensioactif polymérique ayant un HLB compris entre 3 et 7, et ayant un poids moléculaire supérieur à 800 ;
ii) la fourniture d'une base cosmétique de préférence incolore, par exemple blanche ;
iii) la fourniture d'une quantité supplémentaire d'au moins un tensioactif polymérique ayant un HLB compris entre 3 et 7 et ayant un poids moléculaire supérieur à 800 ;
iv) la mise en présence de ladite émulsion intermédiaire de l'étape i), dudit ou desdits tensioactifs de l'étape iii) et

de ladite base cosmétique de l'étape ii) ;
la mise en présence de l'étape iv) pouvant être réalisée en une seule opération, ou par étapes successives, par exemple les étapes ii) et iii) pouvant être réalisées ensemble, de préférence au cours de l'étape iv), d'une part la base cosmétique de l'étape ii) et le ou les tensioactifs de l'étape iii) étant mis en présence puis, d'autre part, l'émulsion intermédiaire étant ajoutée au mélange base cosmétique/tensioactif(s),
dans lequel ledit colorant naturel hydrosoluble ou colorant d'origine naturelle est choisi parmi les anthocyanidines ; les anthocyanines ou les anthocyanes ; les proanthocyanidines ; les proanthocyanines et leurs mélanges.

**15.** Procédé de préparation selon la revendication 14, dans lequel le ou les tensioactifs de l'étape i) et le ou les tensioactifs de l'étape iii) sont différents ou de même nature, de préférence de même nature, et notamment choisis parmi les polyricinoléates de polyglycéryle, les polydialkylsilicones à groupes latéraux et/ou terminaux hydrophiles polyoxyalkylénés, les polymères émulsifiants du type ester d'acide gras de glycol polyoxyalkyléné, et leurs mélanges, plus préférablement choisis parmi les polyricinoléates de polyglycéryle.

**16.** Procédé de préparation d'une composition cosmétique colorée selon l'une quelconque des revendications 1 à 12, comprenant au moins les étapes consistant en :

i) la fourniture d'une émulsion intermédiaire, par exemple de type eau dans huile, comprenant un colorant naturel hydrosoluble ou colorant d'origine naturelle, présent sous forme solubilisée en milieu aqueux et stabilisé par au moins une quantité efficace d'un tensioactif polymérique ayant un HLB compris entre 3 et 7, et ayant un poids moléculaire supérieur à 800, de préférence choisi parmi les polyricinoléates de polyglycéryle, les silicones de polydialkyle à groupements latéraux et/ou terminaux hydrophiles polyoxyalkylénés, et les polymères émulsifiants du type ester d'acide gras et glycol polyoxyalkyléné, et leurs mélanges, plus préférablement choisis parmi les polyricinoléates de polyglycéryle ;
ii) la fourniture d'une base cosmétique de préférence incolore, par exemple blanche ;
iii) la fourniture d'au moins une quantité efficace d'au moins une charge spécifique choisie parmi des particules d'aérogel de silice hydrophobe ;
iv) la mise en présence de ladite émulsion intermédiaire de l'étape i), de ladite ou desdites charges spécifiques de l'étape iii) et de ladite base cosmétique de l'étape ii) ;
la mise en présence de l'étape iv) pouvant être réalisée en une seule opération, ou par étapes successives, par exemple les étapes ii) et iii) pouvant être réalisées ensemble, de préférence au cours de l'étape iv), d'une part la base cosmétique de l'étape ii) et la ou les charges spécifiques de l'étape iii) étant mises en présence puis d'autre part l'émulsion intermédiaire étant ajoutée au mélange base cosmétique/charge(s),
dans lequel ledit colorant naturel hydrosoluble ou colorant d'origine naturelle est choisi parmi les anthocyanidines ; les anthocyanines ou les anthocyanes ; les proanthocyanidines ; les proanthocyanines et leurs mélanges.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 201437006 B
- US 2005019286 A
- US 2013216665 A
- US 7470725 B
- WO 2013190112 A
- WO 2013160362 A
- WO 2013190104 A
- WO 2013194100 A
- US 20050192366 A
- WO 2008155059 A


### Non-patent literature cited in the description


- Cosmetics and Toiletries Magazine. 2005, vol. 120, 10
- *CHEMICAL ABSTRACTS*, 134-01-1
- *CHEMICAL ABSTRACTS*, 134-04-3
- *CHEMICAL ABSTRACTS*, 528-53-0
- *CHEMICAL ABSTRACTS*, 528-28-5
- *CHEMICAL ABSTRACTS*, 643-84-5
- *CHEMICAL ABSTRACTS*, 1429-30-7
- *CHEMICAL ABSTRACTS*, 11029-12-2
- CTFA - International Color Handbook. CTFA, 856-857
- **BRINKER C.J.** ; **SCHERER G.W.** Sol-Gel Science. Academic Press, 1990
- *The Journal of the American Chemical Society*, February 1938, vol. 60, 309
- **VAN DE HULST, H.C.** Light Scattering by Small Particles. Wiley, 1957